# EUROPEAN PATENT APPLICATION

(11) **EP 4 478 052 A1**
(43) Date of publication of application: **18.12.2024**
(21) Application number: 22925248.1
(22) Date of filing: 28.10.2022
(51) Int. Cl.: G01N 35/00

(54) **DATA ANALYSIS METHOD, DATA ANALYSIS SYSTEM, AND COMPUTER**

(30) Priority: 09.02.2022 JP 2022018599
(71) Applicant: HITACHI HIGH-TECH CORPORATION, Tokyo 105-6409 (JP)
(72) Inventor: INOUE, Kohei, Tokyo 105-6409 (JP)
(74) Representative: Strehl Schübel-Hopf & Partner
(86) International application number: PCT/JP2022/040551
(87) International publication number: WO 2023/153032

(57) **Abstract**

Provided is a technology that makes it possible to improve accuracy, etc. when determining the deviation of measurement data from a regression line, etc. and detect abnormalities. According to the present invention, a data analysis method includes a first step in which a computer system acquires reaction process data that includes device information, reagent information, and measurement data from a plurality of automatic analysis systems as reference data, a second step in which the computer system acquires label data that includes, for each piece of reaction process data, an abnormality determination and an abnormality source that have been inputted by an operator, a third step in which, on the basis of an analysis of the reference data and the included label data relative to a distribution map for evaluation parameters for the reaction process data, the computer system calculates overall regression line information 91 that can be commonly applied to the plurality of automatic analysis systems and overall deviation determination reference line information 92 for determining deviation from the overall regression line information 91, and a fourth step in which the computer system displays the overall deviation determination reference line information 92 on a screen 90.

## Description

### Technical Field

The present invention relates to a data analysis technology and in particular to a data analysis technology associated with an automatic analyzer.

### Background Art

An automatic analyzer (also referred to as automatic analysis system) for clinical examination is an apparatus in which certain quantities of a sample and a reagent are dispensed, stirred, and let to react with each other and a liquid obtained as the result of the reaction (also referred to as reaction liquid) is analyzed. Using an automatic analyzer, such a user (also referred to as operator) as a clinical technologist measures an absorbance of a reaction liquid over a certain period of time and determines a concentration of a component to be analyzed based on a result of the measurement. For example, an analysis for clinical examination requires such an apparatus as an automatic analyzer, a reagent for each analysis item, a standard solution for calibrating the reagent, an apparatus under analysis, an accuracy control sample measured for checking a state of the reagent, and the like. Then, other elements than the foregoing are combined to obtain final analysis performance. Examples of the other elements include the dispensing quantity accuracy of the apparatus, the uniformity of the interior of a reagent bottle, storage stability, a degree of chemical reaction, especially, stirring efficiency of the reagent and the sample, a degree of cleaning of a reaction vessel, the stability of the standard liquid, and the like.

As mentioned above, a plurality of factors dominates analysis performance. Elements in the apparatus having influence on analysis performance (in other words, elements having direct influence on analysis performance) include such configuration elements as a sample dispensing mechanism, a reagent dispensing mechanism, a stirring mechanism, an optical system, a reaction vessel, a thermostatic bath, and the like. Other influencing elements than the apparatus include acidity or alkalinity of the reagent, a sample, a control sample, and the like.

As mentioned above, analysis performance is influenced by various elements. For this reason, to use an automatic analyzer, these elements (in other words, influential factors) must be checked to confirm whether a normal clinical examination is feasible.

Calibration processing in an automatic analyzer is performed for each item on a reagent bottle-by-reagent bottle basis using a standard liquid. Specifically, a blank liquid and a standard liquid are measured to determine an origin and an absorbance per unit concentration is computed to compute a conversion factor (also referred to as K factor). In general, a technologist checks a magnitude of absorbance and chronological fluctuation in K factor to determine the acceptability of a calibration result.

After calibration, as accuracy control, an accuracy control sample with a known concentration is measured to check the accuracy based on a difference from a reference value. When a patient sample is measured, ordinarily, an accuracy control sample is regularly measured at certain time intervals to check any deviation from an allowable value. When the allowable value is exceeded, a trouble is considered to have occurred in either a reagent or an apparatus and an inspection is performed. As a confirmation of data in daily examination, reaction process data is used for confirmation. A method for the confirmation differs depending on an analysis method.

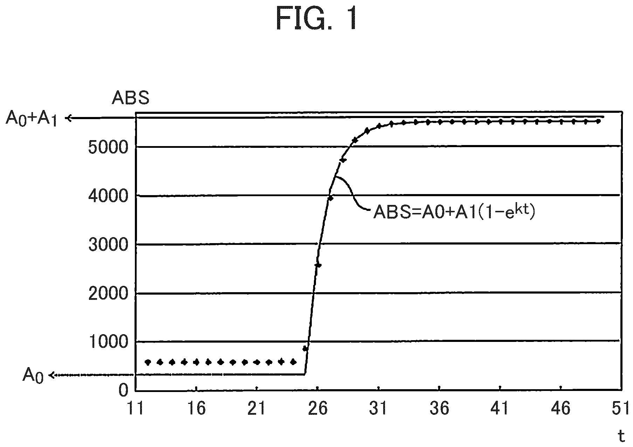

Measurement methos for clinical examination are classified into two types depending on an analysis method: rate method and end point assay. The end point assay relates mainly to methods for measuring such a component as protein and lipoid contained in a sample. In cases where a component in a sample and a reagent react with each other, when a binding reaction is fast, the reaction finishes in a short time and a concentration of a reaction product takes a constant value. When a reaction time is long, it takes some time for a concentration of a reaction product to become constant. When a relation between time and reaction product is plotted in a schematical diagram, the logistic curve (ABS=A0+A1(1-expkt) in FIG. 1 is obtained. k denotes a reaction rate constant. The end point assay is a method for accurately determining a concentration of a measuring object in biochemical analysis. In the end point assay (same as the rate method), data of measured time and absorbance is used to approximate a relation between absorbance and time in a color reaction by a least squares method: y=A+(B-A)/ekt to determine a concentration of a substance to be measured. An example of a conventional method for detecting data abnormality during a measurement by the end point assay is prozone check.

In a reagent using an immuno-nephelometry of, for example, IgA (immune globulin A) or CRP (C reactive protein), protein can precipitate as deposit due to an influence of a salt concentration of a reagent composition. A reaction process can fluctuate due to this deposit and in actuality, this phenomenon often occurs in the latter half of a reaction time. When this fluctuation occurs in a light measurement point portion used in concentration calculation, an accurate measurement value cannot be obtained. Methods for checking this are an antibody readdition method, a ratio of reaction rate method, and the like and any of these methods is to issue an alarm when a limit value specified by parameter is exceeded.

International Publication WO2020/195783 (Patent Literature 1) as a prior art example describes that the accuracy of determination of deviation of measurement data from a regression line is enhanced, that comprehensive regression line information commonly applicable to a plurality of automatic analysis units is generated by a data analysis system based on reference data, and that comprehensive regression line information is displayed in a display screen.

### Citation List

### Patent Literature

Patent Literature 1: International Publication WO2020/195783

### Summary of Invention

### Technical Problem

Owing to the enhancement of the performance of automatic analyzers, even when a trace quantity of a sample or a reagent is used, highly accurate analysis can be performed for various items. Contrarily, sometimes accurate analysis cannot be performed because of a minor trouble in each part of an apparatus, subtle deterioration in the quality of a sample or a reagent. An automatic analyzer for clinical examination measures an absorbance of a reaction liquid, obtained by letting a sample and a reagent react with each other, at certain time intervals as chronological absorbance and measures a rate of change in absorbance and a final absorbance from the obtained chronological absorbance. A concentration of a substance to be measured and an activity value of an enzyme are computed from these pieces of data.

During monitoring of a reaction process, an automatic analyzer performs sample dispensation (sampling), reagent dispensation, stirring, and the like and these processes involve a plurality of error factors, for example, an error due to a difference in the degree of stirring, an error due to a quantity of air bubbles produced during sampling, and the like. Especially, the presence/absence of stirring or a level of stirring cannot be conventionally quantitatively evaluated and criteria are absent. For this reason, an evaluation of the acceptability of reproducibility, the presence/absence of a measurement value obviously involving some trouble, such as discontinuity in measurement value, is obscure. A reagent may be diluted by cleaning water for a reagent probe or a user may erroneously mix a different solution with a reagent; with respect to these factors having direct influence on reaction, an automatic analyzer is required to detect an abnormality and notifies the user of the abnormality to prompt reexamination or maintenance of the apparatus.

It is difficult for a clinical technologist as a user of the automatic analyzer to visually check the entire reaction process during his/her daily examination task and especially, when a measurement value is within a range of normal values, the clinical technologist is prone to overlook a reaction abnormality and this may lead to a result inferior in accuracy.

In Patent Literature 1 as a prior art example, measurement data is utilized to perform analysis but when with respect to determination of deviation from a regression line of measurement data or the like is performed, there is room for enhancement in terms of accuracy and the like.

It is an object of the present invention related to a data analysis technology to provide a technology that, when a determination of deviation from a regression line of measurement data or the like is performed, enables enhancement of accuracy and the like and abnormality detection.

### Solution to Problem

A typical embodiment of the present disclosure includes the configuration elements described below: A data analysis method in the embodiment includes: a first step at which a computer system acquires, as reference data, reaction process data including apparatus information, reagent information, and measurement data from each automatic analysis system of a plurality of automatic analysis systems; a second step at which the computer system acquires label data containing abnormality presence/absence and abnormality cause for each piece of the reaction process data inputted by an operator; and a third step at which with respect to a distribution diagram of evaluation parameters of the reaction process data, based on analysis of the reference data including the label data, the computer system computes comprehensive regression line information commonly applicable to a plurality of the automatic analysis systems and comprehensive deviation determination reference line information for determining deviation from the comprehensive regression line information.

### Advantageous Effects of Invention

According to a typical embodiment of the present disclosure related to a data analysis technology, when determination of deviation from a regression line of measurement data or the like is performed, accuracy and the like can be enhanced and abnormality detection is feasible. Other problems, configuration elements, effects, and the like than described above will be shown in an embodiment for carrying out the present invention.

### Brief Description of Drawings

FIG. 1 is a drawing showing an overview of a logistic curve.
FIG. 2 is a drawing illustrating an example of a configuration of an automatic analysis system in a first embodiment.
FIG. 3 is a drawing illustrating an example of a configuration of an automatic analysis data analysis system in the first embodiment.
FIG. 4 is a drawing illustrating an example of a configuration of an automatic analysis data analysis system in a modification to the first embodiment.
FIG. 5 is a drawing illustrating an example of a configuration of the functional blocks of a comprehensive analysis server in the first embodiment.
FIG. 6 is a drawing illustrating an example of a configuration of the functional blocks of a remote terminal in the first embodiment.
FIG. 7 is a drawing illustrating an example of a configuration of the functional blocks of an automatic analysis system in the first embodiment.
FIG. 8 is a drawing illustrating an example of a configuration of a reference data information table of a remote terminal in the first embodiment.
FIG. 9 is a drawing illustrating an example of a configuration of data organization of a comprehensive analysis server in the first embodiment.
FIG. 10 is a drawing illustrating an example of a configuration of a comprehensive deviation abnormality determination screen displayed in, for example, a remote terminal in the first embodiment.
FIG. 11 is a drawing illustrating a detailed example of GUI in a comprehensive deviation determination screen in the first embodiment.
FIG. 12 is a drawing illustrating another example of a configuration of a comprehensive deviation determination screen in the first embodiment.
FIG. 13 is a drawing illustrating an example of a processing sequence between apparatuses in the first embodiment.
FIG. 14 is a drawing illustrating an example of a processing flow in a comprehensive analysis server in the first embodiment.
FIG. 15 is a drawing illustrating an example of a processing flow with respect to the details of comprehensive analysis processing at Step S1005 in FIG. 14.

### Description of Embodiments

Hereafter, a detailed description will be given to an embodiment of the present disclosure with reference to the drawings. In the drawings, as a rule, an identical part will be marked with an identical sign and a repetitive description thereof will be omitted. In the drawings, an expression of each component may not represent actual position, size, shape, range, or the like for making the present invention understandable. In the drawings, a line representing a communication line or the like is only partly shown but the present invention is not limited to this and, for example, all the components may be connected with one another. A system and the like in the present embodiment may be implemented mainly by software that runs on a versatile computer or may be implemented by dedicated hardware or a combination of software and hardware.

When a description is given to processing by a program, the description may be given mainly to the program, a function, a processing unit, or the like for the sake of convenience; however, a main body as hardware thereof is a processor or a controller, a device, a computer, a system, or the like constituted of the processor and the like. A computer appropriately uses such a resource as a memory or a communication interface by a processor and performs processing in accordance with a program read onto the memory. As a result, a predetermined function, a processing unit, or the like is implemented. A processor is constituted of a semiconductor device and the like, such as CPU, GPU, or the like. Processing need not be software program processing and can also be implemented by a dedicated circuit. FPGA, ASIC, CPLD, and the like are applicable to a dedicated circuit.

A program may be installed in a target computer as data in advance or may be distributed as data from a program source to a target computer. A program source may be a program distribution server on a communication network or may be a non-transient computer-readable storage medium (for example, memory card). A program may be constituted of a plurality of modules. A computer system may be constituted of a plurality of devices. A computer system may be constituted of a client server system, a cloud computing system, an IoT system, or the like.

Varied data and information can be configured in a structure of, for example, table, list, cue, database (DB), or the like but the present invention is not limited to this. For this reason, a table or the like may be simply referred to as information or data. Expressions of identification information, identifier, ID, name, number, and the like can be substituted by one another.

### [Summary and the Like]

The system in Patent Literature 1 of a prior art example utilizes measurement data from an automatic analyzer and such basic information as apparatus information and reagent information to perform analysis (especially, generation of a comprehensive regression line). The system in Patent Literature 1 does not utilize information of the presence/absence of a failure that occurs in an automatic analyzer, the presence/absence of an abnormality in measurement data found by an operation, a cause of an abnormality, or the like (label data in which these pieces of information are recorded) for analysis. As represented by a neural network, in recent years, many effective analysis techniques using label data have been proposed.

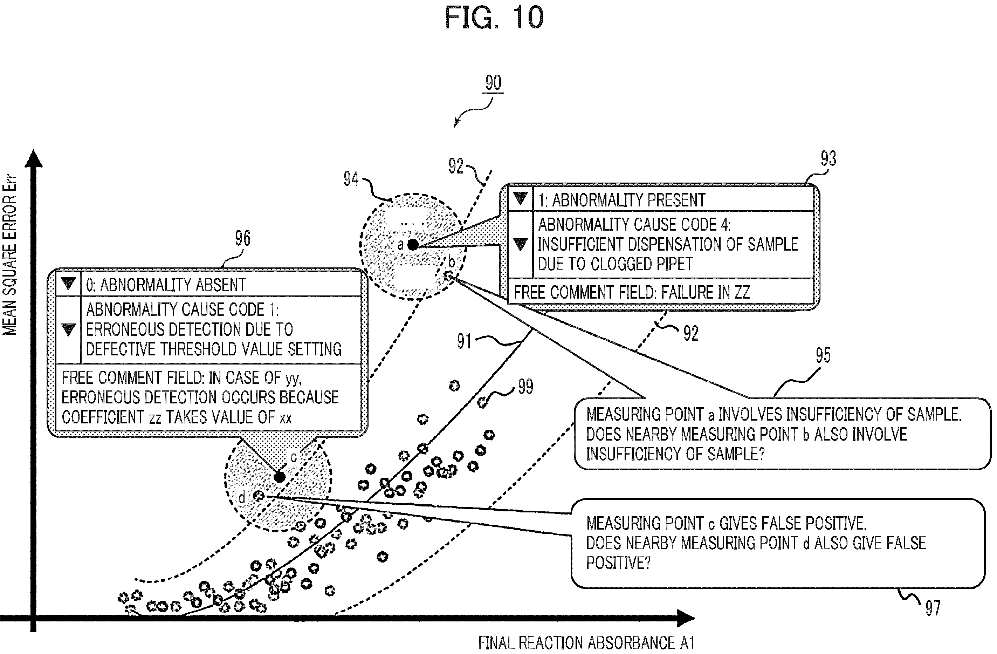

Consequently, the data analysis system in the present embodiment utilizes not only measurement data or basic information but also information of the presence/absence of a failure that occurs in an automatic analyzer, the presence/absence of an abnormality in measurement data found by an operator, a cause of an abnormality, and the like (label data in which these pieces of information are recorded) for analysis. The system in the present embodiment utilizes label data for analysis and leaning and thereby enhances the accuracy of determination of deviation from a regression line (especially, comprehensive regression line) of measurement data and further estimates and detects any abnormality and failure. The data analysis system in the present embodiment effectively acquires label data from an operator. The data analysis system in the present embodiment displays an interface in a display screen for inputting label data and assisting the inputting and displays inputted label data and the like (FIG. 10 and the like described later).

A data analysis method in the present embodiment includes: a first step at which a computer system (for example, a first computer and a second computer) acquires, as reference data, reaction process data including apparatus information, reagent information, and measurement data from each automatic analysis system of a plurality of automatic analysis systems; a second step at which the computer system acquires label data containing the presence/absence of an abnormality and a cause of an abnormality for each piece of reaction process data inputted by an operator; and a third step at which, with respect to a distribution diagram of evaluation parameters of the reaction process data, the computer system computes comprehensive regression line information commonly applicable to a plurality of automatic analysis systems and comprehensive deviation determination reference line information for a determination of deviation from the comprehensive regression line information based on analysis of reference data including label data. Further, the data analysis method includes a fourth step at which the computer system displays comprehensive deviation determination line information in a screen.

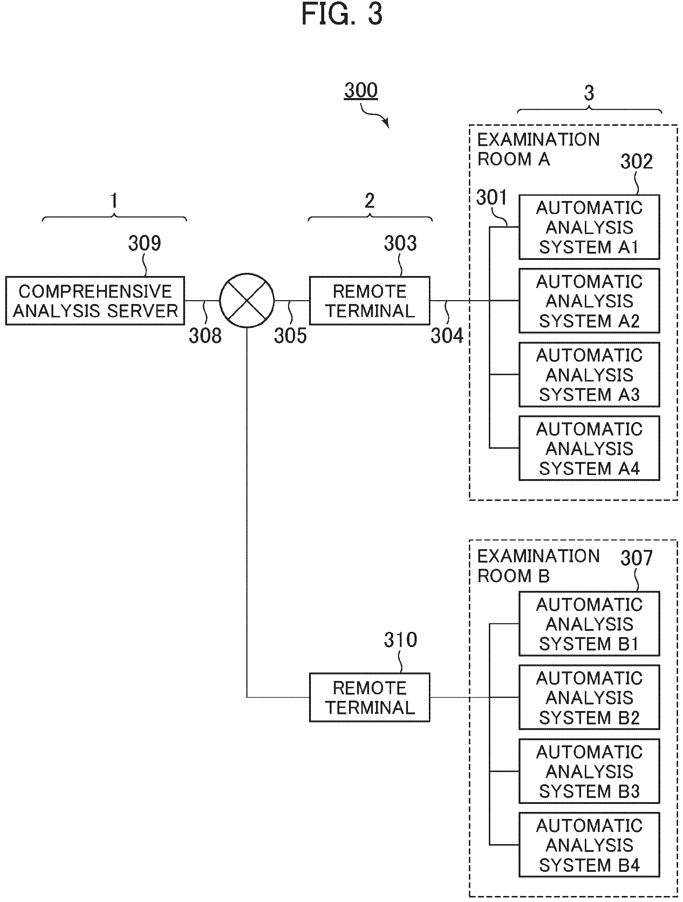

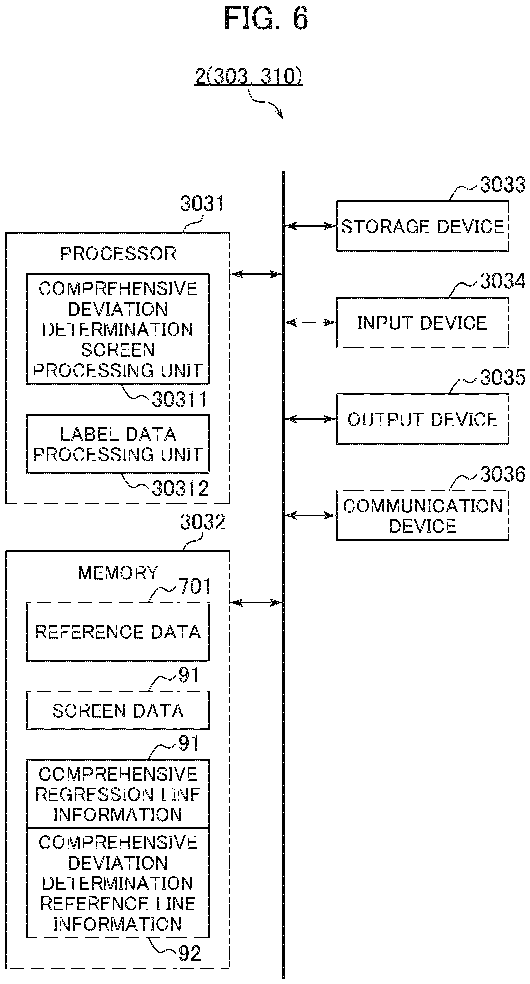

A data analysis method in the present embodiment includes: a step at which each of a plurality of first computers (remote terminals in FIG. 3) acquires information (reference data in FIG. 6) including measurement data and label data from a plurality of automatic analysis systems (automatic analyzer including an automatic analysis unit); a step at which a second computer (comprehensive analysis server in FIG. 3) connected with a plurality of the first computers acquires reference data from a plurality of the first computers; and a step at which the second computer performs analysis (comprehensive analysis) about a plurality of the automatic analysis systems based on reference data and generates analysis result information. The label data is input information such as the presence/absence of an abnormality found or determined by an operator and a cause of an abnormality with respect to reaction process data including measurement data of an automatic analysis system.

The second computer acquires, as reference data, information (referred to as "automatic analysis unit information," "apparatus information," or the like) about an automatic analysis system, information (also referred to as "reagent information") about a reagent and reaction process data including measurement data corresponding thereto through the first computers. Further, the second computer acquires, as a part of reference data, label data inputted by an operator at a display screen of a first computer. Based on these pieces of reference data, the second computer generates and updates comprehensive regression line information commonly applicable to a plurality of automatic analysis units and comprehensive deviation determination reference line information for determining deviation from the comprehensive regression line information, related to an automatic analysis unit and a reagent.

Further, a data analysis method in the present embodiment includes a step at which the second computer sends analysis result information (including comprehensive regression line information and comprehensive deviation determination reference line information) to each of a plurality of the first computers; and each of the first computers acquires the analysis result information and displays, in a display screen, a screen image embracing a distribution diagram of evaluation parameters of reaction process data and comprehensive deviation determination reference line information. A first computer displays, in the screen image, an interface (GUI/screen) for inputting label data about reaction process data and assisting the inputting and acquires label data inputted by an operator through the interface.

The data analysis system (first computer or second computer) compares a point in measurement data in the distribution diagram with the comprehensive deviation determination reference line information, thereby determines the presence/absence of or a degree of deviation from a comprehensive regression line or estimates the possibility of an abnormality, and displays an interface for inputting label data. Based on analysis or learning of the acquired reference data including label data, the second computer updates the comprehensive regression line and the comprehensive deviation determination reference line information.

In the distribution diagram, the data analysis system (second computer or first computer) searches for a point in measurement data at which label data satisfying a predetermined condition has not been inputted as measurement data for which the presence/absence of an abnormality cannot be determined or is difficult to determine. Especially, with respect to a point in first measurement data for which label data has been inputted and considered to be abnormal or in the first measurement data plotted outside the comprehensive deviation determination reference line information, the data analysis system searches for a point in second measurement data for which label data has not been inputted as another measuring point within a nearby region. With respect to the point in the second measurement data, in the screen, the data analysis system displays an interface prompting an operator to input label data in relation with the contents of label data in the first measurement data. This interface can be rephrased as an interface for confirmation or inquiry about the presence/absence of an abnormality, a cause of an abnormality, and the like, presentation of an estimation result, or other like purposes.

### First Embodiment

As an embodiment of the present disclosure, a description will be given to a data analysis system, method, and the like in the first embodiment. As shown in FIG. 3, a data analysis system in the first embodiment is a system including: a plurality of automatic analysis systems (in other words, automatic analyzers provided with an automatic analysis unit); a remote terminal connected thereto; and a comprehensive analysis server connected to the remote terminal. The automatic analysis system or the remote terminal is equivalent to first computer and the comprehensive analysis server is equivalent to second computer. A data analysis method in the first embodiment is a method including steps performed at the first computer or the second computer of the data analysis system in the first embodiment. The computer in the present embodiment is the first computer or the second computer and performs processing in accordance with a program.

In the description of the first embodiment, with respect to an automatic analysis system that quantitively or qualitatively analyzes, for example, a biological sample (also referred to as sample) of blood, urine, and the like, a function of monitoring reaction during a clinical examination/analysis will be mainly taken as an example. Owing to this function, the enhancement of accuracy of a reaction process approximation method and abnormality detection are implemented.

### [Automatic Analysis System]

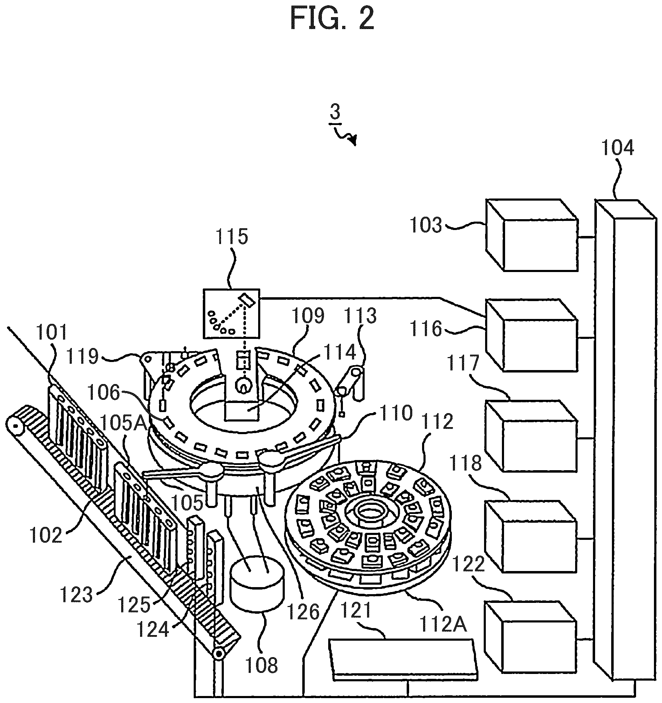

FIG. 2 illustrates a theoretical example of an overall configuration of an automatic analysis system 3, which is an element constituting a data analysis system in the first embodiment. This automatic analysis system 3 is equivalent to one automatic analysis system 302 or the like in FIG. 3 shown later. This automatic analysis system 3 includes a reaction disk 109, a reagent disk 112A, a sample dispensing mechanism 105, a reagent dispensing mechanism 110, a stirring apparatus 113, a cleaning apparatus 119, a light source 114, a multiwavelength photometer 115, a rack conveying apparatus 123, a sample rack 102, an interface 104, a computer 103, and the like.

The reaction disk 109 is a reaction vessel holding mechanism and a plurality of reaction vessels 106 is concentrically set on a circumference. The reaction vessel 106 is a vessel that accommodates reaction liquid. The reaction disk 109 is provided with a rotary drive mechanism, not shown, and is rotatably attached thereto. The reaction disk 109 is kept at a predetermined temperature by a temperature retaining bath 126 connected to a thermostatic bath 108.

The reagent disk 112A is a reagent vessel holding mechanism and a plurality of reagent bins (in other words, reagent bottles, reagent vessels) 112 is concentrically set on a circumference. A plurality of the reagent bins 112 is vessels holding various reagents. The sample dispensing mechanism 105, reagent dispensing mechanism 110, stirring apparatus 113, cleaning apparatus 119, light source 114, and multiwavelength photometer 115 are disposed around the reaction disk 109 and the reagent disk 112A.

A rack conveying apparatus 123 is installed on the rotational circumference of the sample dispensing mechanism 105 and along a tangential direction of the reaction disk 109. A rack number reading apparatus 124 and a sample ID reading apparatus 125 are disposed along a conveyance line. The operations of these mechanisms are all controlled by the computer 103 through the interface 104.

One or more sample vessels 101 holding a sample are set in the sample rack 102. The sample rack 102 is conveyed on the conveyance line by the rack conveying apparatus 123. A serial number is assigned to the individual sample racks 102 and during conveyance on the conveyance line, first, this serial number is read by the rack number reading apparatus 124.

When a respective ID number is assigned to each sample vessel held in the sample rack 102, subsequently, the ID number of a sample is read by the sample ID reading apparatus 125. Thereafter, the sample rack 102 is moved until the first sample vessel 101 held in the rack comes directly under a sample dispensing probe 105A of the sample dispensing mechanism 105. Information read by the rack number reading apparatus 124 and the sample ID reading apparatus is all sent to the computer 103 through the interface 104.

The sample dispensing mechanism 105 dispenses only a predetermined quantity of a sample in a sample vessel 101 into a reaction vessel 106 using the sample dispensing probe 105A under control of the computer 103. After completion of dispensation into one sample vessel 101, the sample rack 102 is so moved that the next sample vessel 101 comes to directly under the sample dispensing probe 105A.

The reaction vessels 106 into which a sample has been dispensed are rotatably moved on the reaction disk 109 by rotational motion of the reaction disk 109. During the foregoing, with respect to a sample in the reaction vessels 106, dispensation of a reagent in a reagent bin 112 by the reagent dispensing mechanism 110, stirring of a reaction liquid by the stirring apparatus 113, and measurement of absorbance by the light source 114 and the multiwavelength photometer 115 are performed. Thereafter, reaction vessels 106 that have undergone analysis are cleaned by the cleaning apparatus 119. An absorbance signal obtained by measurement is inputted to the computer 103 through the interface 104 by way of the A/D converter 116 and is converted there into a concentration of a component to be measured in a sample. Data obtained as the result of concentration conversion is, through the interface 104, displayed in a screen of the CRT (display) 118 or printed out from the printer 117 and is stored in the storage apparatus 122.

The sample dispensing mechanism 105, reaction disk 109, rack conveying apparatus 123, reagent dispensing mechanism 110, stirring apparatus 113, and cleaning apparatus 119 are mechanisms driven by a pulse motor or the like, not shown. Though not shown in the drawing, a plurality of automatic analyzers may also be connected with one another and operated as a single automatic analysis system.

### [Automatic Analysis Data Analysis System]

FIG. 3 illustrates an example of a configuration of an automatic analysis data analysis system 300, which is a system so configured as to include a plurality of automatic analysis systems 3, as a data analysis system in the first embodiment. The automatic analysis data analysis system 300 in FIG. 3 includes, as a plurality of automatic analysis systems 3, a plurality (four in this example) of automatic analysis systems 302 located in a first examination room (Examination Room A) and a plurality (four in this example) of automatic analysis systems 307 located in a second examination room (Examination Room B). The automatic analysis data analysis system 300 includes a remote terminal 303 as first remote terminal and a remote terminal 310 as second remote terminal as two remote terminals 2 connected to a plurality of the automatic analysis systems 3. The automatic analysis data analysis system 300 includes a comprehensive analysis server 309 as a comprehensive analysis server 1 connected to the two remote terminals 2.

The remote terminal 303 is an information processing terminal device connected with a plurality of the automatic analysis systems 302 (A1 to A4 in this example) in Examination Room A through a communication line (for example, LAN) 301 and a communication line 304. Similarly, the remote terminal 310 is an information processing terminal device connected with a plurality of the automatic analysis systems 307 (B1 to B4 in this example) in Examination Room B through communication lines.

The comprehensive analysis server 1 is a server device connected with the remote terminal 303 and the remote terminal 310 through the communication line 305 and the communication line 308. Such a communication means and a communication network as the communication line 308 is, for example, a dedicated line or an internet line.

No limitation is imposed on numbers of the automatic analysis systems 3, remote terminals 2, or comprehensive analysis server 1 shown as an example in FIG. 3. A number of the automatic analysis systems 3 in each examination room is preferably at least one or more and a number of the remote terminals 2 is preferably two or more.

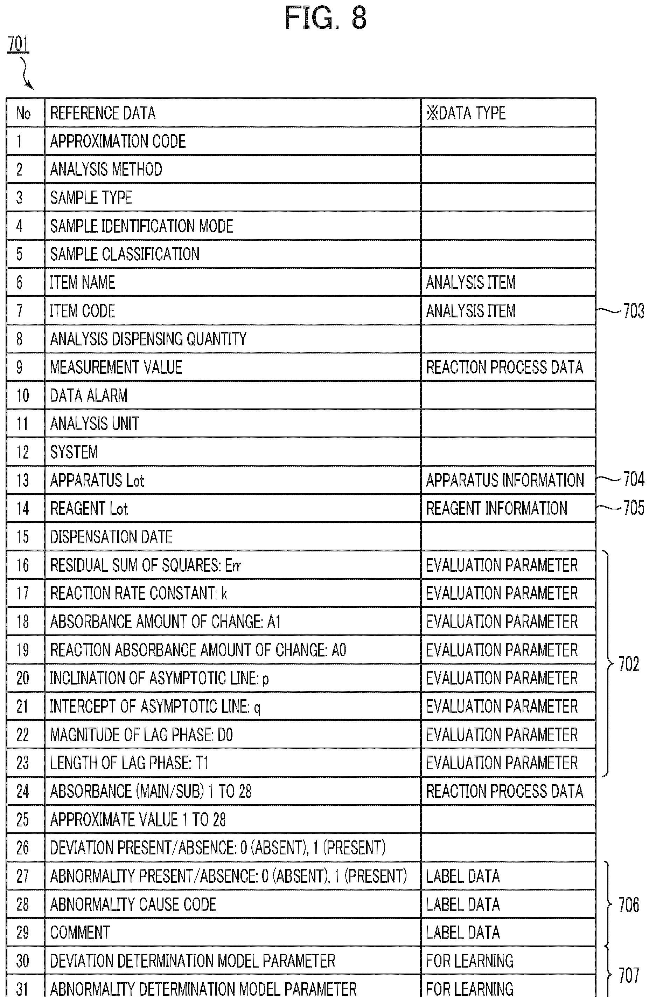

Each automatic analysis system 302 in Examination Room A sends reaction process data including measurement data at an automatic analysis unit to the remote terminal 303. The remote terminal 303 acquires item code, apparatus Lot, reagent Lot, and the like as well from the automatic analysis systems 302. The remote terminal 303 generates reference data (reference data 701 in FIG. 8 shown later) for each automatic analysis system 302 based on the reaction process data received from each automatic analysis system 302 and the like. The remote terminal 303 sends the generated reference data to the comprehensive analysis server 309 through the communication line 305 and the like. Similarly, each automatic analysis system 307 in Examination Room B sends reaction process data including measurement data at an automatic analysis unit to the remote terminal 310. The remote terminal 310 acquires item code, apparatus Lot, reagent Lot, and the like as well from the automatic analysis systems 307. The remote terminal 310 generates reference data for each automatic analysis system 307 based on the reaction process data received from each automatic analysis system 307 and the like. The remote terminal 310 sends the generated reference data to the comprehensive analysis server 309.

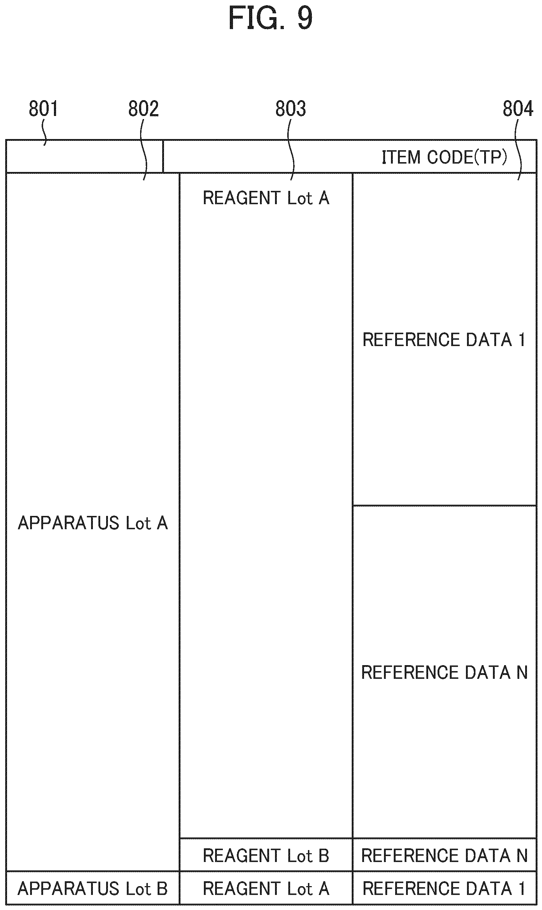

The comprehensive analysis server 1 accumulates information of item code, apparatus Lot, reagent Lot, and the like and reference data including reaction process data acquired from each remote terminal 2 as data by unit of data organization 801 in FIG. 9 shown later. The comprehensive analysis server 1 may acquire reference data from each automatic analysis system 3. Further, the comprehensive analysis server 1 acquires reference data including label data from each remote terminal 2. Using the reference data including the label data, the comprehensive analysis server 1 computes and generates comprehensive regression line information 91 and comprehensive deviation determination reference line information 92 (FIG. 10 shown later), which are information for comprehensive deviation determination and abnormality determination. The comprehensive analysis server 1 sends these pieces of computed information to the remote terminal 301 and the remote terminal 310 (at least either thereof) through the communication line 308 and the like.

With respect to at least one of a plurality of the automatic analysis system 302 provided in Examination Room A, the remote terminal 303 displays, in a display screen, a distribution diagram of reference data (especially, evaluation parameters of reaction process data), the comprehensive regression line information 91, and the comprehensive deviation determination reference line information 92.

Each of the comprehensive analysis server 1 and the remote terminals 2 is configured of a computer system. The computer system includes, for example, a processor, a memory or a storage device, an input device, an output device, a communication device, and the like. The input device and the output device may be an externally connected device or may be an internally mounted device. An external storage device may be provided as the storage device. The memory or the storage device holds various programs, various parameters, and varied data and information. The processor performs processing in accordance with various programs and thereby implements various functions and processing units. The input device is a device for inputting such data information as an instruction and setting from an operator and is, for example, a mouse or a keyboard. The output device is a device for outputting, for example, displaying and printing, a computation result or the like and is, for example, a monitor display, a printer, or the like. The communication device is a device equipped with a communication interface.

Each automatic analysis system 3 is also partly (the computer 103 or the like in FIG. 2) configured of a computer system. A predetermined function of the data analysis system may be implemented by the respective computer systems of the automatic analysis system 3, remote terminals 2, and comprehensive analysis server 1 cooperating with one another by communication. Each computer system may be implemented by communication between a plurality of computers, such as client server systems.

Either or both of the computer 103 of the automatic analysis system 3 and the remote terminal 2 can be rephrased as first computers. The comprehensive analysis server 1 can be rephrased as a second computer. The remote terminals 2 or the comprehensive analysis server 1 is not limited to these names and only has to be a computer system having a predetermined function. For example, the computer 103 of an automatic analysis system 3 can also be so configured as to perform the processing of the first computer and one remote terminal 2 can also be so configured as to perform the processing of the second computer. The remote terminals 2 may be omitted and the automatic analysis data analysis system 300 may be configured of the comprehensive analysis server 1 and a plurality of the automatic analysis systems 3. In other words, the remote terminals 2 and the comprehensive analysis server 1 may be merged into one.

Each automatic analysis system 3 and each remote terminal 2 have a common basic configuration but detailed configuration elements thereof may be different from one another. To the automatic analysis systems 3, remote terminals 2, or comprehensive analysis server 1, as a different apparatus, a mobile information processing terminal apparatus (for example, a tablet terminal or a smartphone) carried by an operator may be connected by communication. A processor of the mobile information processing terminal apparatus may perform the processing of the first computer or the second computer. Each piece of information may be displayed in a display screen of the mobile information processing terminal apparatus.

### [Modification to Data Analysis System]

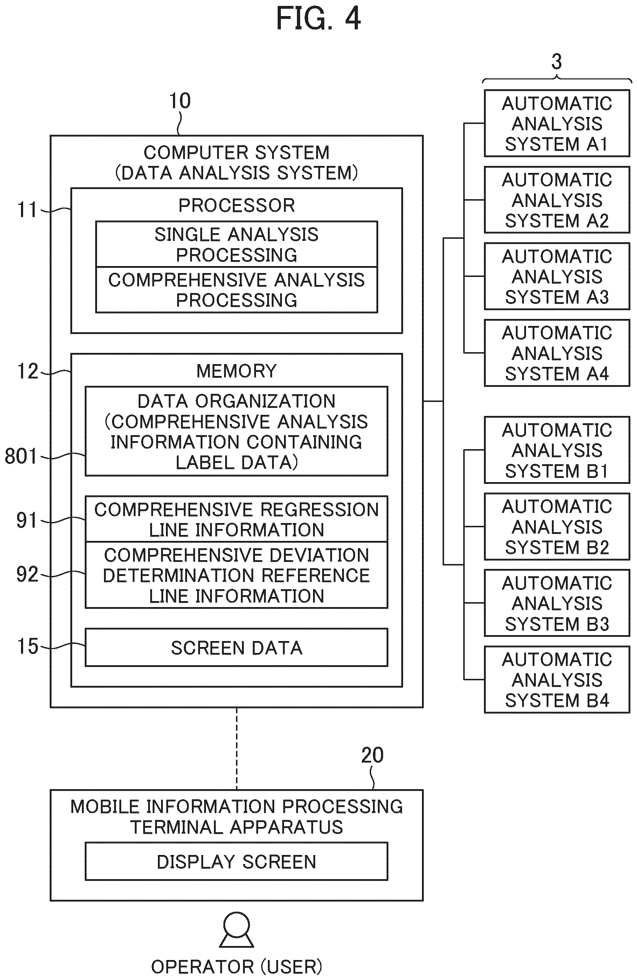

FIG. 4 illustrates a modification to the data analysis system in FIG. 3. The data analysis system in FIG. 4 includes: a data analysis system 10 as a computer system connected to a plurality of automatic analysis systems 3 by communication; and an operator's mobile information processing terminal apparatus 20 connected to the data analysis system 10 by communication. The data analysis system 10 as a computer system includes a processor 11, a memory 12, and the like. The processor 11 performs single analysis processing for each single automatic analysis system 3 and comprehensive analysis processing for a plurality of automatic analysis systems 3. The memory 12 accommodates varied data and information including: comprehensive analysis information containing label data as data organization 801 (FIG. 9); comprehensive regression line information 91 and comprehensive deviation determination reference line information 92 generated as a comprehensive analysis result; and screen data 15 for displaying a comprehensive analysis result and the like.

The data analysis system 10 acquires apparatus information, reaction process data, and the like from each automatic analysis system 3 of a plurality of the automatic analysis systems 3. Further, the data analysis system 10 acquires label data inputted by an operator from each automatic analysis system 3 or the operator's mobile information processing terminal apparatus 20. Examples of the operator's mobile information processing terminal apparatus 20 include a tablet terminal having a display screen based on a touch panel and the like. The mobile information processing terminal apparatus 20 accesses the data analysis system 10 (especially, a server function thereof) based on an operation by an operator. The mobile information processing terminal apparatus 20 acquires screen data 15 containing comprehensive analysis result information from the data analysis system 10 and displays such a comprehensive deviation abnormality determination screen as shown in FIG. 10 in the touch panel display screen thereof based on the screen data 15. An operator inputs label data at the screen. The mobile information processing terminal apparatus 20 sends information containing the inputted label data to the data analysis system 10. Using the label data acquired from the mobile information processing terminal apparatus 20, the data analysis system 10 updates comprehensive deviation determination reference line information 92 or the like.

As another modification, such information as comprehensive deviation determination reference line information 92 is sent from the data analysis system 10 to the mobile information processing terminal apparatus 20 and using the information, the mobile information processing terminal apparatus 20 may generate screen data and display the data in the display screen. The mobile information processing terminal apparatus 20 may estimate and determine the possibility of abnormality based on comparison of a point in measurement data with the comprehensive deviation determination reference line information 92 in the screen.

As another modified system, in such a plurality of remote terminals 2 as shown in FIG. 3, only one specific remote terminal 2 may be provided with a function equivalent to the comprehensive analysis server 1 (second computer) and the one specific remote terminal 2 may appropriately communicate with another remote terminal 2.

### [Comprehensive Analysis Server]

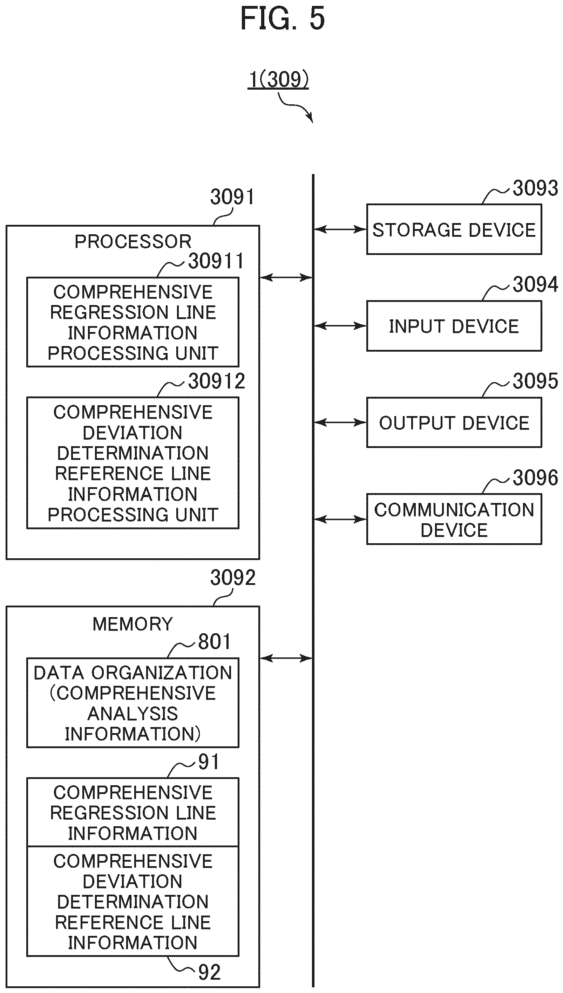

FIG. 5 illustrates an example of a functional block configuration of the comprehensive analysis server 1 (309). The comprehensive analysis server 1 includes, for example, a processor 3091, a memory 3092, a storage device 3093, an input device 3094, an output device 3095, a communication device 3096, and the like. These elements are connected with one another through, for example, a bus.

The processor 3091 performs processing in accordance with various installed programs. The memory 3092 accommodates such data and information as various programs and various parameters. The storage device 3093 accommodates a computation result, acquired information, and the like. The input device 3094 is one or more devices from among a keyboard, a touch panel, various buttons, a microphone, and the like. The output device 3095 is one or more devices from among a display device, a printer, a speaker, and the like. The communication device 3096 is a device connected to a communication line 308 and provided with a communication interface for communication with a plurality of remote terminals 2. In case of a configuration in which the comprehensive analysis server 1 and a plurality of automatic analysis systems 3 are capable of directly communicating with each other, the communication device 3096 has a communication interface for communication with the automatic analysis systems 3.

The processor 3091 reads various programs from the memory 3092 and deploys the programs on a built-in memory, not shown, and appropriately performs program processing. The various programs include a comprehensive regression line information processing program, a comprehensive deviation determination reference line information processing program, and the like. FIG. 5 illustrates how a comprehensive regression line information processing unit 30911 and comprehensive deviation determination reference line information processing unit 30912 implemented by processing in accordance with those programs are implemented in the processor 3091. The comprehensive analysis server 1 includes at least the comprehensive regression line information processing unit 30911 and the comprehensive deviation determination reference line information processing unit 30912. The details of these pieces of processing will be described later:

### [Remote Terminal]

FIG. 6 illustrates an example of a functional block configuration of the remote terminal 2 (303, 310). The two remote terminals 303, 310 are similarly configured; therefore, sometimes, a description will be given only to the remote terminal 303. The remote terminal 2 shown in FIG. 6 includes, for example, a processor 3031, a memory 3032, a storage device 3033, an input device 3034, an output device 3035, and the like.

The processor 3031 performs processing in accordance with various installed programs. The memory 3032 accommodates such data and information as various programs and various parameters. The storage device 3033 accommodates a computation result, acquired information, and the like. The input device 3034 is one or more devices from among a keyboard, a touch panel, various buttons, a microphone, and the like. The output device 3035 is one or more devices from among a display device, a printer, a speaker, and the like. The communication device 3036 is a device connected to the communication line 304 and the communication line 305 and provided with a communication interface for communication with the comprehensive analysis server 1 and a plurality of the automatic analysis systems 3 located in an identical examination room (for example, Examination Room A).

The processor 3031 reads various programs from the memory 3032 and deploys the programs on a built-in memory, not shown, and appropriately performs program processing. The various programs include a comprehensive deviation determination screen processing program, a label data processing program, and the like. FIG. 6 illustrates how a comprehensive deviation determination screen processing unit 30311 and label data processing unit 30312 implemented by processing in accordance with those programs are implemented in the processor 3031. The remote terminal 2 includes at least the comprehensive deviation determination screen processing unit 30311 and the label data processing unit 30312.

The comprehensive deviation determination screen processing unit 30311 of the remote terminal 2 performs processing to display such a comprehensive deviation determination screen as shown in FIG. 10 shown later in the display screen of the display device. At this time, the comprehensive deviation determination screen processing unit 30311 displays, in the comprehensive deviation determination screen, comprehensive regression line information 91 and comprehensive deviation determination reference line information 92 in superimposition on, for example, a distribution diagram of reference data (especially, evaluation parameters of reaction process data) with respect to a plurality of automatic analysis systems 3 provided in an examination room.

Further, in the comprehensive deviation determination screen, the label data processing unit 30312 performs processing to display an interface (GUI/screen) for inputting label data with respect to points in the reaction process data in the distribution diagram and the comprehensive deviation determination reference line information 92 and performs processing to acquire label data inputted by an operator through the interface. At this time, based on comparison of a point in the reaction process data (that is, a measurement result at some automatic analysis system 3) with the comprehensive deviation determination reference line information 92, the label data processing unit 30312 may estimate or determine a degree of deviation with respect to that point or the possibility of abnormality. Based on a result of the estimation or determination, the label data processing unit 30312 may determine whether to display an interface for inputting label data with respect to that point or a type or details of the interface.

The above-mentioned estimation or determination of a degree of deviation or the possibility of abnormality may be performed as a part of comprehensive analysis on the comprehensive analysis server 1 side in advance. In other words, the comprehensive analysis server 1 may be provided with the functions corresponding to the label data processing unit 30312. In this case, comprehensive analysis result information sent from the comprehensive analysis server 1 to the remote terminal 2 may contain information for controlling an interface for inputting label data for each point in measurement data.

### [Computer System of Automatic Analysis System]

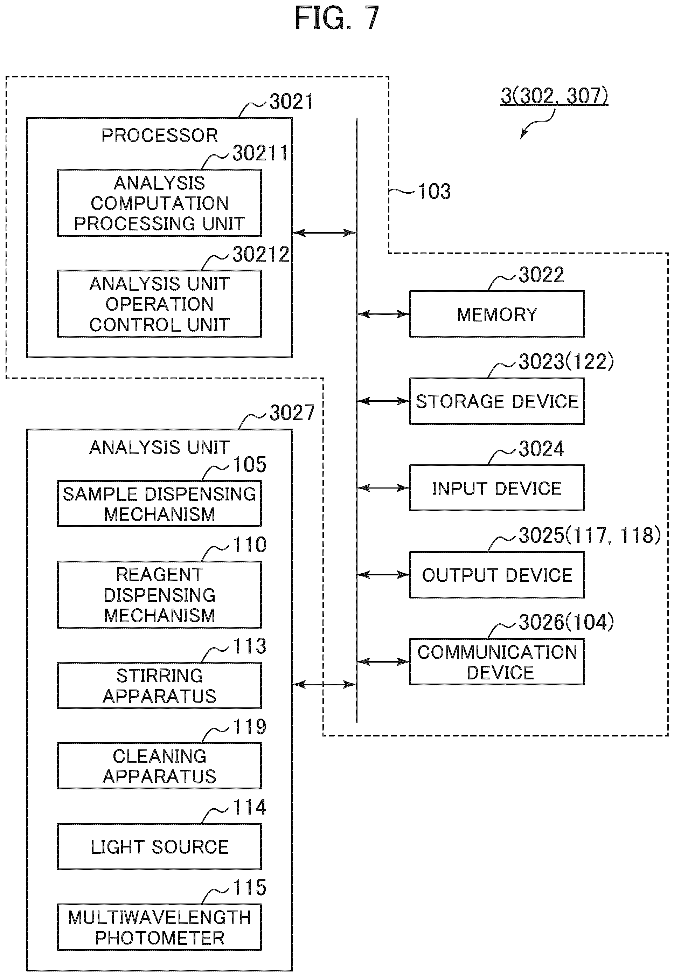

FIG. 7 illustrates an example of a functional block configuration as a computer system of an automatic analysis system 3. The automatic analysis system 3 includes a computer 103 and an analysis unit 3027. The analysis unit 3027 can be rephrased as an automatic analysis unit. The computer 103 includes, for example, a processor 3021, a memory 3022, a storage device 3023, an input device 3024, an output device 3025, a communication device 3026, and the like.

The processor 3021 performs processing in accordance with various installed programs. The memory 3022 accommodates such data and information as various programs and various parameters. The storage device 3023 (equivalent to the storage apparatus 122 in FIG. 2) accommodates a computation result, acquired information, and the like. The input device 3024 is one or more devices from among a keyboard, a touch panel, various buttons, a microphone, and the like. The output device 3025 (equivalent to the printer 117 or the CRT 118 in FIG. 2) is one or more devices from among a display device, a printer, a speaker, and the like. The communication device 3026 is a device connected to the communication line 301 and provided with a communication interface for communication with a corresponding remote terminal 2. In a modification, each automatic analysis system 3 may communicate with other automatic analysis systems in the examination room through the communication device 3026 or may communicate directly with the comprehensive analysis server 1.

The analysis unit 3027 includes such sample dispensing mechanism 105, reagent dispensing mechanism 110, stirring apparatus 113, cleaning apparatus 119, light source 114, multiwavelength photometer 115, and the like as described with reference to FIG. 2.

The processor 3021 reads various programs from the memory 3022 and deploys the programs on a built-in memory, not shown, and appropriately performs processing. The various programs include an analysis computation processing program and an analysis unit operation control program. FIG. 7 illustrates how an analysis computation processing unit 30211 and an analysis unit operation control unit 30212 are implemented in the processor 3021 by processing in accordance with those programs. The analysis computation processing unit 30211 performs, for example, processing to analyze data acquired by the analysis unit 3027. The analysis unit operation control unit 30212 so controls the analysis unit 3027 as to, for example, operate in accordance with an instruction inputted from the input device 3024.

The computer 103 and the analysis unit 3027 may be separated from each other and installed in different places to constitute an automatic analysis system 3. A plurality of analysis units 3027 may be connected to one computer 103 to constitute an automatic analysis system 3.

### [Reference Data]

FIG. 8 indicates an example of organization of a reference data information table (also simply referred to as reference data 701) which is an information table that stores reference data held by each remote terminal 2. Each remote terminal 2 enters information acquired from an automatic analysis system 3 into this reference data information table. The reference data 701 shown in FIG. 8 has a number (No) for each row and a column of reference data and a column of data type is also shown for making the explanation understandable.

The reference data 701 in FIG. 8 contains the following items in the numerical order:
- 1.: approximation code,
- 2.: analysis method,
- 3.: sample type,
- 4.: sample identification mode,
- 5.: sample classification,
- 6.: item name,
- 7.: item code,
- 8.: analysis dispensing quantity,
- 9.: measurement value,
- 10.: data alarm,
- 11.: analysis unit,
- 12.: system,
- 13.: apparatus Lot,
- 14.: reagent Lot,
- 15.: dispensation date,
- 16.: residual sum of squares: Err,
- 17.: reaction rate constant: k,
- 18.: absorbance amount of change: Al,
- 19.: reaction absorbance amount of change: A0
- 20.: inclination of asymptotic line: p,
- 21.: intercept of asymptotic line: q
- 22.: magnitude of lag phase: D0,
- 23.: length of lag phase: T1,
- 24.: absorbance (main/sub) 1 to 28,
- 25.: approximate value 1 to 28,
- 26.: deviation present/absent: 0 (absent), 1 (present),
- 27.: abnormality present/absent: 0 (absent), 1 (present),
- 28.: abnormality cause code,
- 29.: comment,
- 30.: deviation determination model parameter,
- 31.: abnormality determination model parameter.

The reference data 701 in FIG. 8 is generated by entering reaction process data and the like including measurement data, sent from an automatic analysis system 3. The reference data 701 contains, as constituent information, item code 703, apparatus Lot 704, reagent Lot 705, evaluation parameter 702, label data 706, and model parameter 707.

The reaction process data including measurement data especially refers to data containing a measurement value of 9, absorbance of 24, and the like of the reference data 701. The item code 703 is a code for identifying an analysis item (or examination item) assigned to a reagent. In cases where a plurality of chemical manufacturers exists with respect to item code 703, even reagents of the same type may be different from manufacturer to manufacturer; therefore, different codes are assigned thereto. The apparatus Lot 704 is apparatus information (in other words, automatic analysis unit information) indicating an apparatus name, a production unit, and the like of an automatic analysis unit of an automatic analysis system 3. The reagent Lot 705 is reagent information indicating a production unit of a reagent.

The approximation code of 1 is a code for identifying an approximate expression of a reaction process. A combination of an approximate expression and an evaluation parameter to be used is selected according to a combination of an analysis item (item code) and a reagent (reagent information). Correlation among those pieces of information may be stored in a table in advance. The data alarm of 10 is alarm information added and outputted as data when an automatic analysis system detects various errors or the like.

The evaluation parameter 702 is a parameter for evaluating a reaction process of measurement data and includes a parameter and the like constituting an approximate expression of a reaction process. In the present example, the evaluation parameter 702 includes a plurality of parameters from the residual sum of squares Err of 16 to the length of lag phase T1 of 23. A description will be given to an example of the evaluation parameter 702. The absorbance amount of change A1 of 18 (in other words, final reaction absorbance) is an absorbance amount of change of reaction process data in the end point assay. For example, when x=a0-al*exp(-kt) is used as an approximate expression, an absorbance amount of change A1 becomes identical with a parameter a1 of the approximate expression. The residual sum of squares Err of 16 is a mean square error of a difference between an absorbance approximate value computed by an approximate expression and an actually measured absorbance measured value at each time. For example, these two evaluation parameters (A1, ERR) can be taken as two axes to generate such a distribution diagram as shown in FIG. 10 shown later.

Further, in the rate method, for example, evaluation parameters showing a curved shape representing a reaction process are provided. A time period from start of a reaction is taken as the horizontal axis and absorbance is taken as the vertical axis; then an approximate curve of change in absorbance determined by an approximate expression can be obtained. With respect to the approximate curve, an asymptotic line can be obtained. Time at which the approximate curve is asymptotically brought close to the asymptotic line is computed and a time period from 0 to that time (in other words, lag time) is equivalent to the length of a lag phase T1. The inclination and intercept of the asymptotic line are equivalent to the parameters of 20 and 21. The present invention is not limited to the above-mentioned example of evaluation parameters, which can be arbitrarily defined.

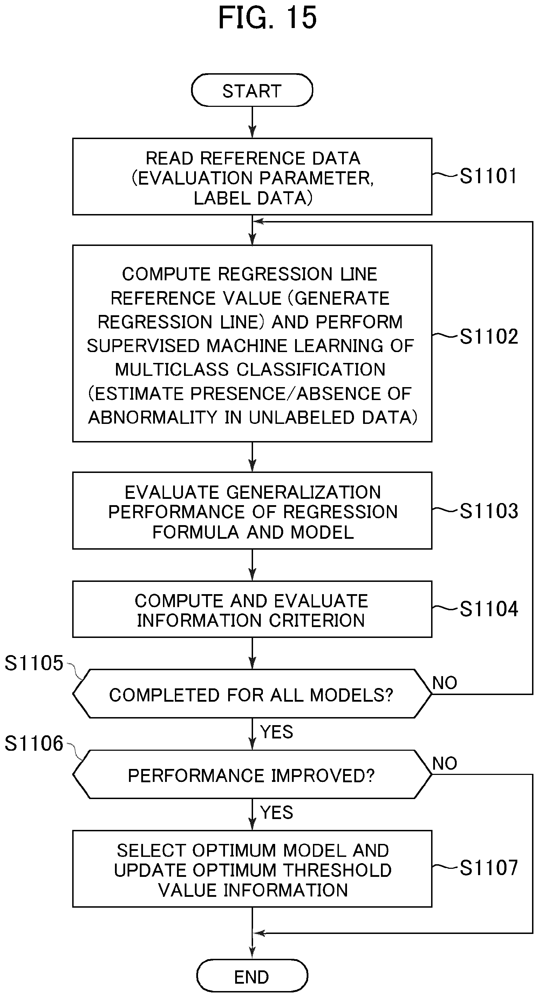

The label data 706 includes abnormality present/absent of 27, abnormality cause code of 28, and comment of 29. The label data 706 is data inputted mainly by an operator. The data analysis system defines option information of the label data 706 in advance and provides the information in such a screen as shown in FIG. 10 shown later so that an operator can select and input the information. The label data 706 is used in supervised machine learning (FIG. 15), described later, using the label data as teacher information.

The label data 706 in FIG. 8 is an example in which information inputted mainly by an operator is entered but the present invention is not limited to this and information of the presence/absence of a failure that has occurred in an automatic analysis system 3 may be included as a part of the label data 706. For example, when any other apparatus, such as an automatic analysis system 3 or a remote terminal 2, detects the presence/absence of a failure in an automatic analysis system 3, information of the presence/absence of the failure may be outputted to, for example, a remote terminal 2 and the remote terminal 2 may enter the information of the presence/absence of the failure into the reference data as a part of the label data. Alternatively, an operator may input information of the presence/absence of a failure in an automatic analysis system 3 as a part of the label data at a screen of a remote termina 2 or the like.

The deviation present/absent of 26 is information indicating a result of a determination of the presence/absence of deviation from the comprehensive regression line information 91 made by a computer system (first computer or second computer) using the comprehensive deviation determination reference line information 92. This deviation present/absent of 26 may be so configured that the information can be inputted by an operator as a part of the label data 706.

The model parameter 707 is parameter information of a computation model used in the learning (FIG. 15), described later, using the reference data 701 including the label data 706. An example of the parameter information is parameter information constituting a model (CNN) of a neural network. Aside from learning, parameter information constituting an algorithm for processing of deviation determination or the like may also be applied to the model parameter 707. Initially, an initial value of the model parameter 707 is generated by the comprehensive analysis server 1. The comprehensive analysis server 1 updates the model parameter 707 according to learning as appropriate.

### [Example of Data Organization of Comprehensive Analysis Server]

FIG. 9 illustrates an example of a configuration of data organization 801 so configured as to include a reference data information table accumulated in the comprehensive analysis server 1. The data in the data organization 801 is all the data acquired from the remote terminal 2 (303, 310) side and held by the comprehensive analysis server 1 and is, in other words, comprehensive analysis data. The comprehensive analysis server 1 acquires such reference data 701 as shown in FIG. 8 from each remote terminal 2 and generates and holds data in the data organization 801 containing these pieces of data. Varied data may be managed using a file system, a database, or the like.

In the example of data organization 801 in FIG. 9, reference data 1 to N is shown as a plurality of pieces of reference data and the reference data 1 to N is, for example, data of each sample. Each of these pieces of reference data contains varied data and information containing label data 706 or a model parameter 707 as new data elements, as shown in FIG. 8 shown above.

The data organization 801 stores and manages each piece of reference data 804 corresponding to each reagent Lot 803, for example, for each apparatus Lot 802. In FIG. 9, the data organization 801 is configured in layers of apparatus Lot 802 and reagent Lot 803 for each item code 703 in the reference data 701 in FIG. 8. The present invention is not limited to this example of data organization as long as a link is established between pieces of information of apparatus Lot 802 and the like.

### [Comprehensive Regression Line Information and Comprehensive Deviation Determination Reference Line Information]

Comprehensive regression line information 91 generated by the comprehensive analysis server 1 through comprehensive analysis is information of a regression line and the like generated by totalizing reference data (including especially reaction process data and evaluation parameters) of a plurality of automatic analysis systems 3 as targets to which the information is commonly applied. The comprehensive deviation determination reference line information 92 generated by the comprehensive analysis server 1 is information of a reference line that provides a threshold value for determination of a degree of deviation from the comprehensive regression line information 91. A degree of deviation is more increased as a point of a combination of evaluation parameters of reaction process data is more brought away from the comprehensive regression line information 91. Especially, when a relevant point is plotted beyond and outside the comprehensive deviation determination reference line information 92, it is estimated that the relevant reaction process data is highly possibly abnormal.

### [Example of Comprehensive Deviation Determination Screen]

FIG. 10 illustrates an example of a configuration of a screen (also referred to as comprehensive deviation abnormality determination screen 90) displayed in a display screen of a display device of a remote terminal 2 (or an automatic analysis system 3) based on analysis result information from the comprehensive analysis server 1. This screen 90 embraces a distribution diagram of evaluation parameters of reaction process data, comprehensive regression line information 91, and comprehensive deviation determination reference line information 92. In the present example, this screen 90 is displayed in a display screen of the remote terminal 303.

The screen 90 in FIG. 10 embraces a distribution diagram of combinations of evaluation parameters 702 of reaction process data as a distribution diagram of reference data. In the present example, this distribution diagram is a distribution diagram in which as an example of the evaluation parameters 702, final reaction absorbance A1 (absorbance amount of change A1 of 18 in FIG. 8) is taken on the horizontal axis and mean square error Err (residual sum of squares Err of 16 in FIG. 8) is taken on the vertical axis. Each point in measurement data 99 and the like is a plot of each piece of reference data (in other words, measurement data, reaction process data). Each point can be differently displayed in, for example, different colors, shapes, or the like for each target automatic analysis system 3 or sample. In the present example, a combination of A1 and Err is taken as an example of a combination of evaluation parameters 702 but the present invention is not limited to this and a multidimensional distribution diagram based on a combination of two or more evaluation parameters 702 is similarly possible.

A plurality of points is plotted in the distribution diagram and these point groups are an example obtained by using history data groups within a specified period with respect to one or more samples in one or more automatic analysis systems 3. When data is plotted with respect to a plurality of samples, each point can be differently displayed in different colors or shapes. For example, data of a first sample is plotted as green points and data of a second sample is plotted as blue points. As another example, in a distribution diagram, for example, a data group from Examination Room A may be displayed in green and a data group from Examination Room B may be displayed in blue.

Not only in the example in FIG. 10, at least comprehensive deviation determination reference line information 92 is displayed in a display screen of a remote terminal 2. Though in the example in FIG. 10, comprehensive regression line information 91 is also displayed, the display of the comprehensive regression line information 91 may be omitted.

In the comprehensive deviation abnormality determination screen 90 in FIG. 10, the comprehensive regression line information 91 and the comprehensive deviation determination reference line information 92 are displayed in superimposition on the distribution diagram of reference data (especially, combinations of evaluation parameters of reaction process data). The comprehensive regression line information 91 indicated by solid line indicates a comprehensive regression line (in other words, regression function) with respect to measurement data group obtained from a plurality of automatic analysis systems as targets indicated by the point groups. The comprehensive deviation determination reference line information 92 indicated by broken line is reference lines for a determination of deviation from the comprehensive regression line information 91 and, in other words, indicates a border line on both sides constituting a reference range and lines constituting a threshold value; and in the present example, the reference line is two curved lines established on both sides of the comprehensive regression line information 91. The comprehensive deviation determination reference line information 92 can be rephrased as comprehensive abnormality detection information for comprehensively detecting any abnormality with a plurality of automatic analysis systems 3 taken into account.

The data analysis system (may be the comprehensive analysis server 1 or may be a remote terminal 2; for example, the comprehensive analysis server 1) can estimate a point plotted within a range between the two curved lines of the comprehensive deviation determination reference line information 92 to be not deviated and can estimate a point plotted out of the range to be deviated. For example, such measurement data as point a indicated by filled circuit located outside one (diagonally left upward in the drawing) reference line is estimated to be data involving deviation, in other words, abnormal data.

When an operator performs an operation to specify a point in measurement data, detailed information about reaction process data and reference data including measurement data corresponding to the specified point, for example, information of a curved line or the like representing the reaction process may be displayed in a screen (for example, pop-up screen). A regression line and a deviation determination reference line can also be displayed with respect to each single automatic analysis system 3 and this is described in Patent Literature 1.

In the screen 90 in FIG. 10, with respect to the measurement data (abnormal data) of point a involving deviation, a GUI/scree (also referred to as label data input screen 93) that enables label data to be inputted is displayed. This label data input screen 93 is a GUI/screen at which an operator can input label data (label data 706 in FIG. 8) containing abnormality present/absent, abnormality cause code, and comment. This label data input screen 93 may be a GUI part displayed in superimposition on a distribution diagram of reference data, the comprehensive regression line information 91, or the like as shown in the drawing, may be a multiwindow or a different screen that separately makes a transition, or may be a different GUI part that is not displayed in a superimposed manner but is displayed in parallel or in any other like manner. In the present example, with respect to the measurement data of point a, the label data input screen 93 is displayed as a speech bubble-like GUI part. The label data input screen 93 embraces such GUI parts as list box, text box, and the like.

Further, the data analysis system (may be the comprehensive analysis server 1 or may be a remote terminal 2; for example, the comprehensive analysis server 1) computes a nearby region 94 (shown by broken line circle) with respect to the measurement data of point a, which is abnormal data involving deviation. The data analysis system detects measurement data that is plotted within the nearby region 94 of point a and located inside the comprehensive deviation determination reference lines 92 and for which label data has not been inputted. In the present example, the measurement data of point b is measurement data that satisfies those conditions.

Examples of methods for defining the nearby region 93 include a method by clustering and a method of making definition by L1 norm, L2 norm, Mahalanobis distance, or the like. The nearby region 94 is configured as a partial space within a multidimensional space obtained by combining evaluation parameters 702 in FIG. 8. That is, the nearby region 94 in the present example is obtained by plotting a projection of a partial region defined in multidimensions onto a two-dimensional plane. In the present example, the nearby region 94 is a circle within a range at a predetermined distance with point a at the center and is a two-dimensional region.

The data analysis system (may be the comprehensive analysis server 1 or may be a remote terminal 2; for example, the comprehensive analysis server 1), based on the nearby region 94, with respect to the measurement data of point b, displays a GUI/screen (also referred to as label data input prompting screen 95) that prompts an operator to additionally input label data. With respect to the relevant measurement data, the label data input prompting screen 95 may be a GUI part displayed in a superimposed manner as shown in the drawing or may be a different GUI part or the like displayed not in a superimposed manner but in parallel or in any other like manner. In the present example, with respect to the measurement data of point b, the label data input prompting screen 95 is displayed as a speech bubble-like GUI part. The label data input prompting screen 95 embraces a text message prompting input of label data with respect to point b.

Similarly, in the screen 90, point c is an example of measurement data (abnormal data) plotted outside the comprehensive deviation determination reference line information 92 and involving deviation. With respect to point c, a label data input screen 96 is displayed. Point d is measurement data that is plotted within a nearby region of point c and located outside the comprehensive deviation determination reference lines 92 and for which label data has not been inputted. With respect to such measurement data as point d, the data analysis system displays a label data input prompting screen 97.

In the screen 90 in FIG. 10, with respect to points in each piece of measurement data, like a, b or the like in the drawing, such information as identification sign may be added to display. Also, with respect to such a nearby region as the nearby region 94, like the broken line circle in the drawing, information indicating a nearby region may be displayed. When a cursor is brought close to a point or an operation is performed to select a point, a nearby region and other points embraced in the nearby region may be highlighted.

The details of the screen 90 in FIG. 10 are as described below: In a modification, the screen 90 may be similarly displayed in a display screen of a display device of each automatic analysis system 3. As in FIG. 4 shown above, the screen may be similarly displayed in a display screen of a data analysis system 10 or an operator's mobile information processing terminal apparatus 20.

In relation with the comprehensive deviation determination reference line information 92, each point in measurement data may be displayed in different colors, shapes, or the like based on a deviation determination by the data analysis system. For example, since point a is located outside the comprehensive deviation determination reference line information 92, the data analysis system estimates the point to involve deviation and be abnormal and the point may be displayed in predetermined color or shape (for example, in red circle). With respect to a point plotted inside the comprehensive deviation determination reference line information 92 and located within a predetermined distance from the comprehensive deviation determination reference line information 92, the data analysis system may estimate the point to be possibly abnormal and the point may be displayed in predetermined color or shape. Point b is located in the nearby region 94 of point a and plotted inside the comprehensive deviation determination reference line information 92; with respect to such a point, the data analysis system may estimate this point to be possibly abnormal in relation to point a which is abnormal data and may display the point in predetermined color or shape (for example, in orange circle) .

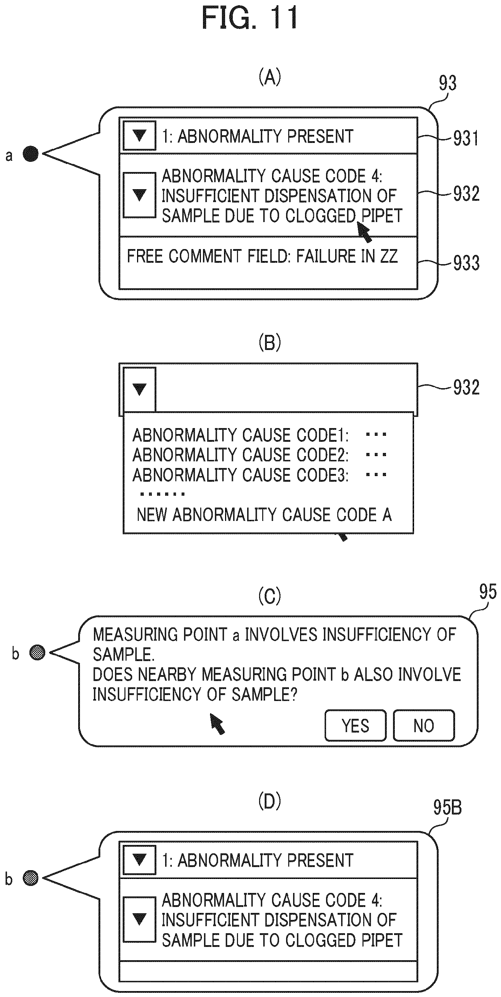

FIG. 11 shows the details of the screen 90 in FIG. 10. (A) in FIG. 11 shows a label data input screen 93 for point a and embraces an abnormality present/absent field 931 for inputting the presence/absence of an abnormality, an abnormality cause field 932 for inputting an abnormality cause, and a comment field 933 for inputting a comment. The abnormality present/absent field 931 is comprised of a list box and an operator can selectively input "abnormality present" or "abnormality absent." In the present example, the abnormality present/absent field 931 involves two options, abnormality present and absent but in a modification, three or more options may be provided. For example, a value for abnormality undeterminable (cases where the presence/absence of an abnormality cannot be determined or is difficult to determine) may be added or, a degree or the possibility of an abnormality may be classified into three or more values. The abnormality cause field 932 is comprised of a list box and an operator can selectively input from among a plurality of abnormality cause codes specified as options in advance. For example, "abnormality cause code 4: insufficient dispensation of sample due to clogged pipet" is selected. The comment field 933 is comprised of a text box and an operator can freely input a comment in text. In the comment field 933, for example, "failure in ZZ" is inputted. An example of a comment is supplementation about an abnormality cause or the like.

Initially, information determined by the data analysis system may be displayed in the label data input screen 93 as default information (in other words, initial value). For example, when point a is estimated or determined to be abnormal, "1: Abnormality present" is displayed as an initial value in the abnormality present/absent field 931 in the label data input screen 93. An operator can visually check the initial value and keep the initial value unchanged when the initial value is appropriate and select and input "0: Abnormality absent" when it is determined that an abnormality is absent.

When the data analysis system can automatically determine an abnormality cause, the abnormality cause information obtained by the automatic determination may be automatically displayed as an initial value in the abnormality cause field 932. For example, when an abnormality cause code or a data alarm of 10 in FIG. 8 is outputted from an automatic analysis system 3, a remote terminal 2 or the comprehensive analysis server 1 may estimate or determine an abnormality cause based on the outputted information. An operator can keep the initial value unchanged when the initial value is appropriate and selectively input any other abnormality cause code when it is determined that the abnormality is due to any other cause.

When an operator suspects any other abnormality cause than the abnormality cause codes prepared as options in advance, the operator can also generate a new abnormality cause code. The data analysis system has a function for this purpose. (B) in FIG. 11 illustrates an example in which a new abnormality cause code is inputted in the abnormality cause field 932. An operator performs operation to select the item of "new abnormal cause code A", rather than the existing abnormality cause codes, from among options displayed in the list box in the abnormal cause field 932. The indication of this item may also be "generate new abnormality cause" or the like. The operator generates, inputs, and registers the contents of "new abnormality cause code A" in text in the abnormality cause field 932. A register button or a cancel button may be separately displayed. After this new registration, the newly registered "new abnormality cause code A" is displayed as one option in the list box in the abnormality cause field 932; therefore, an operator can utilize the new abnormality cause code again.

Varied information inputted by an operator through a GUI part at the screen 90 in FIG. 10 is automatically stored and updated as data by background processing. Or, when an enter button or the like is provided in the screen 90 and the enter button or the like is inputted, corresponding data may be stored or updated. A remote terminal 2 may communicate with the comprehensive analysis server 1 as appropriate and may send input data at the screen 90 or updated reference data 701 (including label data 706) to the comprehensive analysis server 1.

(C) in FIG. 11 illustrates the details of a label data input prompting screen 95 with respect to point b. In this label data input prompting screen 95, as a message example, "Measuring point a involves insufficiency of sample. Does nearby measuring point b also involve insufficiency of sample?" is displayed. In consideration of the contents of label data inputted at the label data input screen 93 for point a, the data analysis system automatically displays a label data input prompting screen 95 for point b located inside the comprehensive deviation determination reference line information 92. With respect to measurement data of point a, the information of insufficient dispensation of sample of "abnormality cause code 4" has been inputted as label data. Based on the foregoing, the data analysis system can presume the possibility that the abnormality cause of point b within the nearby region 94 of point a is also identical with that of point a.

Consequently, in the label data input prompting screen 95 for point b, the data analysis system communicates a message telling the possibility that the abnormality cause of nearby point b is identical with that of point a and prompts input of label data. As in the above example, with respect to such measurement data as point b located inside the comprehensive deviation determination reference line information 92, it is more preferable to take into account label data of point a (point in relation of location within the nearby region 94) located outside the comprehensive deviation determination reference line information 92 to generate the contents of the label data input prompting screen 95.

An operator can visually check the contents of the label data input prompting screen 95, determine an abnormality cause of point b, and correspondingly make an affirmative or a negative input. In the present example, when an operator puts a cursor within the label data input prompting screen 95, the YES/NO buttons, shown in the drawing, are displayed. When the contents of the message are correct, that is, it is determined that an abnormality cause estimated by the data analysis system is proper, the operator presses the YES button; and when not correct, that is, it is determined that an abnormality cause is different, the operator presses the NO button. When either button is pressed, a label data input screen for point b is displayed.

First, when the YES button is pressed, such a label data input screen 95B as in (D) is displayed. In this label data input screen 95B, the same contents as such label data input screen 93 (especially, the abnormality present/absent field 931 and the abnormality cause field 932) of point a as in (A) is automatically displayed as an initial value. Also, with respect to the comment field 933, the same contents may be displayed. An operator can visually check the contents of the label data input screen 95B for point b and can keep the initial value unchanged when the initial value is proper and correspondingly input different abnormality present/absent and an abnormality cause in the abnormality present/absent field and the abnormality cause field when it is determined that the presence/absence of the abnormality or the abnormality cause is different from those of point a.

When the NO button is pressed, a label data input screen different in contents from the label data input screen 93 for point a and having a predetermined initial value (for example, null is also acceptable) is automatically displayed. The operator inputs information determined by him/herself into each field in the label data input screen.

The present invention is not limited to the above-mentioned YES/NO buttons and any other GUI is also acceptable. For example, to cope with cases where YES/NO determination cannot be made, an undeterminable button or the like may be provided. A label data input screen displayed in correspondence with the undeterminable button or the like may be so configured that information indicating that an abnormality cause or the like is undeterminable or unknown can be inputted.

As mentioned above, with respect to such measurement data as point b for which label data has not been inputted, GUI is provided in relation to nearby point a and auxiliary information is provided for determining the presence/absence of an abnormality and an abnormality cause; therefore, time and effort for label data input work by an operator can be reduced. The data analysis system can generally and efficiently acquire label data with respect to a plurality of pieces of measurement data.

Also, with respect to point c and point d in FIG. 10, the case is substantially the same as the above examples of point a and pint b but a case of false positive will be described as a difference. As in the label data input screen 93 for point a, in the label data input screen 96 for point c, the abnormality present/absent field, the abnormality cause field, and the comment field are displayed. An operator inputs information into each field in the label data input screen 96. For example, in the abnormality present/absent field, "0: Abnormality absent" is inputted. Though point c is located outside the comprehensive deviation determination reference line information 92, the operator determines that an abnormality is absent (in other words, erroneous detection by the system, false positive) and selectively input "0: Abnormality absent." In the abnormality cause field, "Abnormality cause code 1: Erroneous detection due to defective threshold value setting" is selectively inputted. In the comment field, for example, "In case of yy, erroneous detection occurs because coefficient zz takes a value of xx" is inputted. The threshold value cited here corresponds also to the comprehensive deviation determination reference line information 92; when the current comprehensive deviation determination reference line information 92 is updated to be widened outward, point c is determined to be normal.

Point d is located in the nearby region of point c and is plotted outside the comprehensive deviation determination reference line information 92 and label data has not been inputted therefor. Since like point a and point c, point d is located outside the comprehensive deviation determination reference line information 92, point d can be simply determined to be abnormal; but the data analysis system further makes a determination in relation to point c, which is in neighboring relation. As a result of determination in consideration of the contents (information of erroneous detection or false positive) of label data for nearby point c, with respect to point d, the data analysis system can estimate the possibility of erroneous detection or false positive. Consequently, with respect to point d, the data analysis system automatically displays a label data input prompting screen 97. The label data input prompting screen 97 contains, for example, a message of "Measuring point c gives false positive. Does nearby measuring point d also give false positive?" The operator can visually check the contents of the label data input prompting screen 97 and, as in the example of the label data input prompting screen 95, correspondingly make an affirmative/negative input as required. For example, the YES button is pressed in the label data input prompting screen 97, a label data input screen identical in contents with that for point c is displayed. The operator can check the label data input screen and appropriately correct the contents and input the corrected contents.

As in the above-mentioned example, in the screen 90 in FIG. 10, as a basic function, an operator can input label data at GUI/screen for each point in each piece of measurement data. In the first embodiment, further, as a more intelligent function, the data analysis system automatically makes estimation or determination and presents an initial value for label data input, a prompt to input label data, and information of the presence/absence of an abnormality, a result of estimation of an abnormality cause, and the like. As a result, with respect to a larger number of pieces of measurement data, an operator can easily perform label data input work through a screen.

As another modification to the screen 90 in FIG. 10, with respect to each point in each piece of measurement data, a label data input screen and a label data input prompting screen may be integrated into one to display. With respect to a plurality of points in a distribution diagram, a display may be so made that whether label data has not been inputted or has been already inputted can be discriminated. When an operation is made to select a point for which label data has been already inputted, in a displayed label data input screen, an operator can confirm the already inputted label data and correct the label data as required.

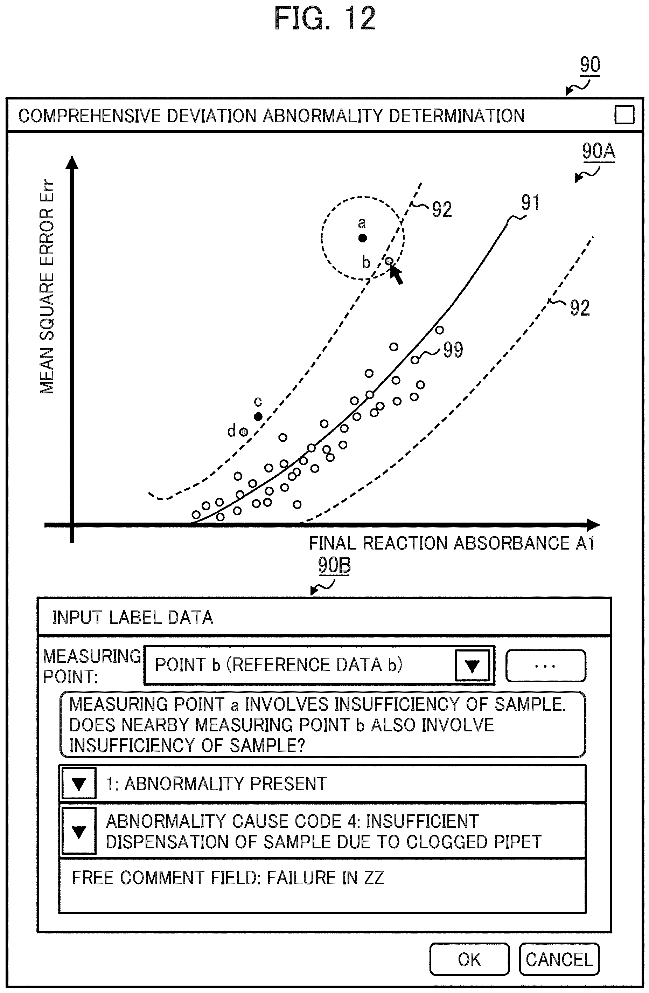

FIG. 12 illustrates an example of display of the screen 90 in a modification. In the present example, the screen 90 is a window corresponding to a Web page. In this screen 90, aside from the same distribution diagram 90A (details omitted) as in FIG. 10, a label data input field 90B is displayed in parallel. The label data input field 90B embraces an item for displaying a target measurement point (reference data corresponding to the measuring point; for example, a point specified by a cursor in the distribution diagram 90A) in a selectable manner, a label data input prompting message, and the same abnormality present/absent field, abnormality cause field, and comment field as mentioned above.

To such display of the comprehensive deviation determination screen 90 as in FIG. 10, not only display in each remote terminal 2 or each automatic analysis system 3 but also display in any computer or the like related thereto may be similarly applied. Such screen 90 as in FIG. 10 may be generated and displayed based on analysis result data that a remote terminal 2 received from the comprehensive analysis server 1. Alternatively, screen data in a mode of Web page or the like having such contents as in the screen 90 may be sent from the comprehensive analysis server 1 to a remote terminal 2 and the remote terminal 2 may directly display the screen data in the mode of Web page or the like.

### [Processing Sequence]

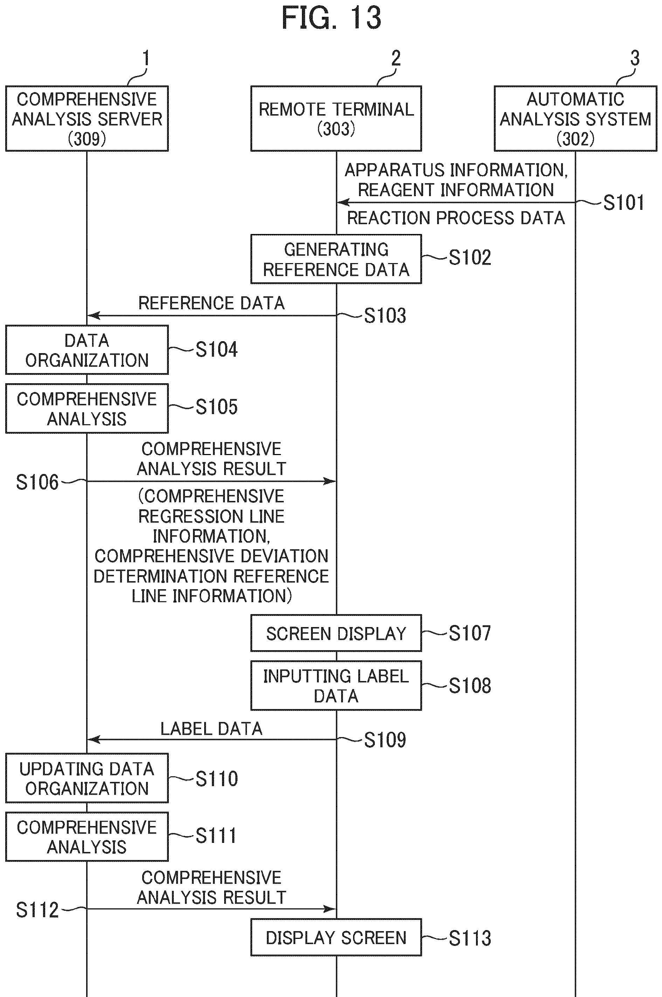

FIG. 13 illustrates an example of a processing sequence in the automatic analysis data analysis system 300 in FIG. 3. At Step S101, each automatic analysis system 3, for example, the automatic analysis system 302 in Examination Room A sends apparatus information, reagent information, and reaction process data to a remote terminal 2 (for example, the remote terminal 303). At Step S102, based on reaction process data or the like acquired from each automatic analysis system 3, the remote terminal 2 generates such reference data 701 (however, initially, reference data not having label data 706 or the like) as in FIG. 8. At Step S103, the remote terminal 2 sends reference data of each automatic analysis system 3 or examination room to the comprehensive analysis server 1 (309).

At Step S104, based on reference data acquired from each remote terminal 2, the comprehensive analysis server 1 generates data (comprehensive analysis data) in such data organization 801 as in FIG. 9 and holds the data. As Step S105, based on the data in the data organization 801, the comprehensive analysis server 1 performs comprehensive analysis for common application to a plurality of target automatic analysis systems 3 and obtains comprehensive analysis result information and holds the information. This comprehensive analysis processing includes comprehensive regression line information processing to compute comprehensive regression line information 91 and comprehensive deviation determination reference line information processing to compute comprehensive deviation determination reference line information 92. At Step S106, the comprehensive analysis server 1 sends information including the comprehensive regression line information 91 and the comprehensive deviation determination reference line information 92 to the target remote terminal 2 as comprehensive analysis result information.

At Step S107, based on the comprehensive analysis result information received from the comprehensive analysis server 1, the remote terminal 2 displays such a screen (comprehensive deviation abnormality determination screen 90) as in FIG. 10 toward the operator. In this screen, such interfaces as the above-mentioned label data input screen 93 are also automatically displayed. As Step S108, the operator views the screen to confirm a distribution diagram of reaction process data, the comprehensive regression line information 91, the comprehensive deviation determination reference line information 92, and the like. Further, according to the label data input screen 93 or the label data input prompting screen 95, the operator inputs or corrects label data at the screen as appropriate. At Step S109, the remote terminal 2 enters the label data inputted at the screen into the reference data 701 in FIG. 8 and sends reference data (in other words, updated reference data) including the label data to the comprehensive analysis server 1. In a modification, only inputted label data may be sent from the remote terminal 2 to the comprehensive analysis server 1.

As Step S110, based on the reference data including the label data acquired from the remote terminal 2, the comprehensive analysis server 1 updates data in such data organization 801 as in FIG. 9. As Step S111, based on the data in the updated data organization 801, the comprehensive analysis server 1 performs comprehensive analysis using the label data and holds comprehensive analysis result information. At Step S112, the comprehensive analysis server 1 sends the comprehensive analysis result information obtained at Step Sill to the remote terminal 2. At Step S113, based on the comprehensive analysis result information from the comprehensive analysis server 1, the remote terminal 2 displays a screen with the contents updated as in FIG. 10. The steps from label data input of Step S108 can be similarly repeated as required.

Like the system in Patent Literature 1, the data analysis system in the first embodiment performs comprehensive deviation determination and the like based on comprehensive regression line information 91 with a plurality of automatic analysis systems targeted. In deviation determination based only on reference data of a single automatic analysis system, the accuracy of the deviation determination can be insufficient because of, for example, bias of sample, machine difference, or the like. For this reason, the data analysis system in the first embodiment generates comprehensive regression line information 91 and comprehensive deviation determination reference line information 92 based on reference data of a plurality of automatic analysis systems 3 and performs comprehensive deviation determination and the like based on those pieces of information. At this time, the data analysis system in the first embodiment uses acquired label data to perform comprehensive analysis and updates the comprehensive deviation determination reference line information 92 and the like.

As in the system in Patent Literature 1, in the data analysis system in the first embodiment, for example, a remote terminal 2 can perform evaluation processing itself of reaction process for each single automatic analysis system 3. Meanwhile, the comprehensive analysis server 1 comprehensively performs evaluation processing of reaction process for a plurality of automatic analysis systems 3 in a lump based on reference data as a processing result of each remote terminal 2. As a result of that processing, the comprehensive analysis server 1 adjusts an evaluation parameter 701, a model parameter 707 for learning, and the like. The present invention is not limited to the foregoing and in a modification, for example, the comprehensive analysis server 1 (in the case of FIG. 4, the data analysis system 10) may perform all the evaluation and analysis processing, including single analysis and comprehensive analysis.

### [Processing by Comprehensive Analysis Server]

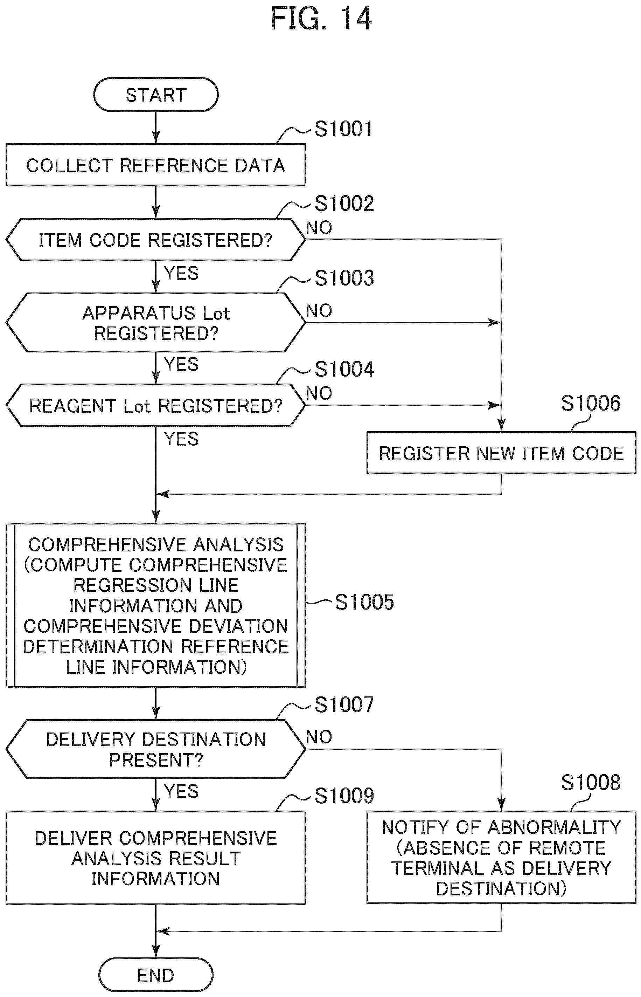

FIG. 14 illustrates a flow of processing by the comprehensive analysis server 1 (second computer) in the data analysis system in the first embodiment. The processing in this flow is implemented mainly by software program processing by, for example, the processor 3091 in FIG. 5.

When periodically communicating with each remote terminal 2 (Step S103 in FIG. 13), at Step S1001, the comprehensive analysis server 1 starts the comprehensive regression line information processing unit 30911, comprehensive deviation determination reference line information processing unit 30912 in FIG. 5, and the like. Periodically cited here refers to, for example, such timing as once per day or each time a predetermined number of pieces (for example, 1000 pieces) of new reference data is accumulated. Communication connection between the remote terminal 2 and the comprehensive analysis server 1 may be triggered from the remote terminal 2 side or from the comprehensive analysis server 1 side.

The processor of the comprehensive analysis server 1 collects reference data from the remote terminal 2. Specifically, the processor acquires such reference data 701 as in FIG. 8 from, for example, the connected remote terminal 303. The reference data 701 contains item code 703, apparatus Lot 704 indicating an automatic analysis system 302 or the like connected to the relevant remote terminal 303, reagent Lot 705 of a reagent used in the relevant automatic analysis system 302 or the like, and the like.

At Step S1002, the processor of the comprehensive analysis server 1 checks item code 703 contained in the acquired reference data. Specifically, the processor checks whether the item code 703 has been registered in data in data organization 801 of the comprehensive analysis server 1. When the item code 703 has been registered in the data organization 801 (S1002: YES), the processing proceeds to Step S1003; when not (S1002: NO), the processing proceeds to Step S1006.

At Step S1003, with respect to the already registered item code 703, the processor of the comprehensive analysis server 1 checks whether apparatus Lot 704 has been registered in data in the data organization 801. When the apparats Lot has been already registered (S1003: YES), the processing proceeds to Step S1004; when not (S1003: NO), the processing proceeds to Step S1006.

At Step S1004, with respect to the already registered apparatus Lot 704, the processor of the comprehensive analysis server 1 checks whether reagent Lot 705 has been registered in data in the data organization 801. When the reagent Lot 705 has been already registered (S1004: YES), the processing proceeds to Step S1005; when not (S1004: NO), the processing proceeds to Step S1006.

At Step S1005, the processor of the comprehensive analysis server 1 adds the reference data 701 acquired from the remote terminal 2 to reference data 804 in data in the data organization 801. Based on the data organization 801, the processor of the comprehensive analysis server 1 generates comprehensive regression line information 91 and comprehensive deviation determination reference line information 92. Specifically, using the reference data 1 to N in FIG. 9, the processor generates the comprehensive regression line information 91 and the comprehensive deviation determination reference line information 92 for each item code 703, apparatus Lot 803, and reagent Lot 804. The details of processing to compute these pieces of information will be described later with reference to FIG. 15. The processor stores the computed comprehensive regression line information 91 and comprehensive deviation determination reference line information 92 in the storage device 3093 or the like.

At Step S1006, the processor of the comprehensive analysis server 1 newly adds and registers information of item code 703, apparatus Lot 704, and reagent Lot 705 that has not registered in the data organization 801 to the data organization 801 as new item codes.

At Step S1007, with respect to comprehensive analysis result information including the comprehensive regression line information 91 and comprehensive deviation determination reference line information 92 computed at Step S1005, the processor of the comprehensive analysis server 1 checks whether a remote terminal 2 as a destination of delivery is present. The presence/absence of a remote terminal 2 as a destination of delivery is determined, for example, according to whether apparatus Lot 803 and reagent Lot 804 as original data for generating the relevant comprehensive deviation determination reference line information 92 or the like have been registered in the remote terminal 2 (303 or 310) connected to the comprehensive analysis server 1.

When it is determined that a remote terminal 2 as a destination of delivery is absent (S1007: NO), the processing proceeds to Step S1008; when it is determined that a remote terminal 2 as a destination of delivery is present (S1007: YES), the processing proceeds to Step S1009. There are also cases where apparatus Lot 803 and reagent Lot 804 for generating the comprehensive deviation determination reference line information 92 or the like have been registered in the data organization 801 but a remote terminal 2 as a destination of delivery may be absent. An example of this case is a case where a relevant reagent corresponding to reagent Lot registered in the data organization 801 was used before in an automatic analysis system 3 of the relevant apparatus Lot but the relevant reagent is not used at the present. Or, an example of this case is a case where an automatic analysis system 3 corresponding to the above-mentioned apparatus Lot 803 has been absent among the automatic analysis systems 3 presently managed by a remote terminal 2. The check at Step S1007 is useful as a countermeasure against cases where a reagent or the like that had been used before is not used at the present.

At Step S1008, the processor of the comprehensive analysis server 1 displays notification of abnormality (absence of a remote terminal 2 as a destination of delivery) in, for example, a display screen of the display device as the output device 3095 in FIG. 5 and terminates the flow in FIG. 14.

At Step S1009, the processor of the comprehensive analysis server 1 delivers comprehensive analysis result information including the comprehensive regression line information 91 and comprehensive deviation determination reference line information 92 generated at Step S1005 to the remote terminal 2 as a destination of delivery and terminates the flow in FIG. 14. After reception of the comprehensive analysis result information from the comprehensive analysis server 1, based on the comprehensive analysis result information, the remote terminal 2 displays the above-mentioned comprehensive deviation determination screen or performs label data input processing. Further, based on the comprehensive analysis result information, the remote terminal 2 may perform deviation determination or abnormality detection processing.

With respect to the delivery destination check at Step S1007, when a new automatic analysis system 3 is added into, for example, the system in FIG. 3 as a new delivery destination, such processing as that in FIG. 14 can be similarly performed for the new automatic analysis system 3. When the type (apparatus Lot) of the newly added automatic analysis system 3 or an applied reagent (reagent Lot) is identical with those in an existing automatic analysis system group, existing comprehensive analysis result information is also applicable to the newly added automatic analysis system 3.

### [Details of Comprehensive Analysis Processing]

FIG. 15 illustrates a detailed flow of processing with respect to the comprehensive analysis processing (processing to compute the comprehensive regression line information 91 and the comprehensive deviation determination reference line information 92) at Step S1005 in FIG. 14. This comprehensive analysis processing is performed, for example, when a sufficient amount of reference data is collected at the above-mentioned Step S1001 and accumulation is completed. A sufficient amount cited here refers to an amount necessary and sufficient for learning or the like.

The reference data used at Step S1005 is divided into that used for learning at Step S1102 and that used for evaluation at Step S1103 or S1104. Examples of methods for dividing reference data include holdout validation, k-cross validation, and the like. The evaluation parameters 702 and the like are considered to be identical in the divided two pieces of data (in other words, two sets of data groups) and different measurement data groups are used for learning and for evaluation. For example, two sets of data groups may be generated by selecting a plurality of points at random from reference data groups related to combinations (for example, a set of A1 and Err) of identical evaluation parameters 702. A first set of a data group is for learning and a second set of a data group is for evaluation.

As Steps S1101, the processor of the comprehensive analysis server 1 reads reference data. This operation includes acquisition of the evaluation parameters 702 and label data 706 of all the reference data (FIG. 8) that has been accumulated. Further, as mentioned above, the processor divides the read reference data into data for learning at Step S1102 and data for evaluation at Step S1103.

At Step S1102, first, the processor performs regression formula reference value computation processing (in other words, regression line generation processing) using evaluation parameters 702 in FIG. 8 and expresses a distribution of combinations (for example, sets of A1 and Err) of evaluation parameters 702 in a regression formula. This regression formula reference value computation processing is performed without fail. For regression formula expressing a distribution of evaluation parameters 702, for example, five types which are 0th order function, linear function, quadratic function, logarithmic function, and exponential function, are used.

Each function provides the regression formula described below: X and Y cited here denote a combination of evaluation parameters. Ck (k=1 to 4) indicates a coefficient of a regression formula.

| | |
|---|---|
| 0th order function: | Y = C1 |
| Linear function: | Y = C1X + C2 |
| Quadratic function: | Y = C1X² + C2X + C3 |
| Logarithmic function: | Y = C1 log (C3X + C4) + C2 |
| Exponential function: | Y = C1 exp (C3X + C4) + C2 |

The computed regression formulas are equivalent to the comprehensive regression line information 91 (FIG. 10).

In cases where setting to perform learning is established, at Step S1102, learning is performed in addition to the above-mentioned generation of regression formulas. Whether to perform learning and a learning method can be specified by system setting or user setting by an operator. To perform this learning, evaluation parameters 702 and label data 706 in FIG. 8 are used. In the example in the first embodiment, this learning is supervised machine learning that performs a multiclass classification task. The reason why the multiclass classification task is performed is that a plurality of abnormality causes can be present. To this supervised machine learning, for example, multiclass logistic regression analysis, a multiclass neural network, or the like is applicable. The processor uses data (also referred to as labeled data) with a label (label data 706) affixed thereto as teacher information to perform this machine learning. Using this learning, the processor estimates the presence/absence of an abnormality and an abnormality cause with respect to data (also referred to as unlabeled data) without a label (label data 706) affixed thereto.

When labeled data available to the above-mentioned supervised machine learning is insufficient, the processor may use clustering (for example, k-means method, k-means++, or the like) to simulatively affix a label to unlabeled data in proximity to labeled data to perform supervised machine learning.

During the learning, the processor may utilize a model parameter 707 in FIG. 8 as an initial parameter of a computation model for learning. The model parameter 707 is parameter information applied to a computation model for learning. The deviation determination model parameter of 30 is parameter information of a model for determining deviation from comprehensive regression line information 91. The abnormality determination model parameter of 31 is parameter information of a model for determining any abnormality based on comprehensive deviation determination reference line information 92.

When learning is performed at Step S1102, generalization performance evaluation at Step S1103 is also performed. At Step S1103, to evaluate the regression formula (in other words, regression line, regression function, especially, comprehensive regression line information 91) generated at Step S1102 and a multiclass classification model, the processor of the comprehensive analysis server 1 evaluates the generalization performance of the multiclass classification model using labeled data. The labeled data used in this evaluation is data for evaluation (the above-mentioned divided other data) that was not used for learning at Step S1102.

Examples of methods for generalization evaluation include F value, AUC, drawing of a ROC curve, or a combination thereof, and the like. The comprehensive regression line information 91 that provides a reference for deviation and the comprehensive deviation determination reference line information 92 for determining the presence/absence of an abnormality are adopted as numerical values that increase the generalization performance to the highest.

At Step S1104, the processor of the comprehensive analysis server 1 computes and evaluates an information criterion for preventing overfit of the above-mentioned model. Examples of methods for computing an information criterion include AIC and BIC.

At Step S1105, with respect to all the models involved in the above-mentioned learning and evaluation, the processor determines whether computation has been completed. When the computation has been completed (S1105: YES), the processing proceeds to Step S1106; when not (S1105: NO), the processing proceeds to Step S1102 and the steps S1102 to S1104 are similarly repeated.

At Step S1106, the processor confirms whether the performance has been improved in all the models. For example, the processor determines whether the generalization performance and the information criterion of a model is more excellent than those of models that have ever been applied. When the performance has been improved (S1106: YES), the processor proceeds to Step S1107; when not (S1106: NO), for example, when the generalization performance and information criterion of a model are inferior to models that have ever been applied, the processor terminates the flow. At Step S1107, the processor selects a predetermined number (for example, an optimal one) of most suitable or favorable models from among models involving performance improvement. For example, the processor selects an optimal model high in generalization performance and low in information criterion and updates a model for learning and evaluation to the relevant model. The processor adopts the comprehensive regression line information 91 tailored to the updated optimal model and the comprehensive deviation determination reference line information 92 that provides optimum threshold value information as updated information.

### [Effects and the Like]

As described up to this point, according to the data analysis method and the like in the first embodiment, when determination of deviation from a regression line of measurement data of an automatic analysis system or the like are performed, accuracy and the like can be enhanced and abnormality detection is enabled. Especially, according to the first embodiment, when determination of deviation from the comprehensive regression line information 91, abnormality detection, or the like is performed with respect to a plurality of automatic analysis systems 3, accuracy and the like can be enhanced.

(1) According to the first embodiment, the comprehensive analysis server 1 as a second computer can acquire label data that is not conventionally utilized from a remote terminal 2 as a first computer and can generate and update favorable comprehensive regression line information 91 and comprehensive deviation determination reference line information 92 by machine learning and analysis using the label data. As a result, accuracy related to comprehensive deviation determination and the like with a plurality of automatic analysis systems 3 targeted can be enhanced more than conventional art.
(2) According to the first embodiment, label data can be efficiently acquired from an operator through such a screen as in FIG. 10. A computer system (first computer and second computer) provides a function of enabling label data containing the presence/absence of an abnormality and an abnormality cause to be inputted by an operator at a screen. As that function, the computer system provides a screen embracing GUI for inputting label data to an operator. Especially, the computer system estimates the presence/absence of an abnormality or the like with respect to measurement data plotted in proximity to comprehensive deviation determination reference line information 92 in the screen and provides a label data input screen or a label data input prompting screen with respect to measurement data estimated to be abnormal. Especially, in relation to first measurement data for which label data has been inputted and which is considered to be abnormal, the computer system provides a label data input prompting screen for prompting, confirming, and inquiring about label data input with respect to second measurement data which is located within the nearby region and for which label data has not been inputted and which is estimated to be abnormal. Owing to the foregoing, acquisition of label data can be made more efficient.

The functions and the like described in relation to a data analysis system and method in the first embodiment can also be implemented by a program (in other words, a program code of software) and a storage medium with the program recorded thereon. The program can be described in various programming languages, scripts, or the like, such as assembler, C/C++, Perl, and the like. The program or the storage medium is provided to a computer system in the present embodiment. The processor of the computer system reads a program stored in, for example, a storage medium onto a memory and performs processing in accordance with the program. As a result, the functions and the like in the present embodiment are implemented. Examples of applicable storage media include flexible disk, CD-ROM, DVD-ROM, hard disk, optical disk, magneto-optical disk, CD-R, magnetic tape, nonvolatile memory card, ROM, and the like.

The technology of the embodiments of the present disclosure can be implemented utilizing various versatile devices (for example, PC or server on a communication network) without being limited to a specific device or can also be implemented in a dedicated device (for example, a part of an automatic analysis system). The functions and the like of the present embodiment may be implemented by an operating system (OS) or middleware running on the computer system performing a part of actual processing based on an instruction from a program.

Up to this point, a concrete description has been given to an embodiment of the present disclosure but the present invention is not limited to the embodiment of the disclosure and can be variously modified without departing from the subject matter of the present invention. With the respect to the present embodiment, a component can be added, deleted, or substituted except an indispensable component. Unless specially limited, each component may be single or multiple. A mode in which various configuration examples are combined is also acceptable.

### List of Reference Signs

90... screen (comprehensive deviation abnormality determination screen),
91... comprehensive regression line information,
92... comprehensive deviation determination reference line information,
93... label data input screen,
94... nearby region,
95... label data input prompting screen,
96... label data input screen,
97... label data input prompting screen,
99... measurement data.

## Claims

1. A data analysis method comprising:
a first step at which a computer system acquires, as reference data, apparatus information, reagent information, and reaction process data including measurement data from each automatic analysis system of a plurality of automatic analysis systems;
a second step at which the computer system acquires label data containing the presence/absence of an abnormality and an abnormality cause for each piece of the reaction process data inputted by an operator; and
a third step at which with respect to a distribution diagram of evaluation parameters of the reaction process data, based on analysis of the reference data including the label data, the computer system computes a comprehensive regression line information commonly applicable to a plurality of the automatic analysis systems and comprehensive deviation determination reference line information for determining deviation from the comprehensive regression line information.

2. The data analysis method according to Claim 1, comprising:
a fourth step at which the computer system displays the comprehensive deviation determination reference line information in a screen.

3. The data analysis method according to Claim 1, comprising:
a step of, based on analysis of the reference data that does not include the label data, the computer system generating first comprehensive regression line information and first comprehensive deviation determination reference line information as the comprehensive regression line information and the comprehensive deviation determination reference line information;
a step of the computer system displaying a screen embracing a distribution diagram of evaluation parameters of the reaction process data and the first comprehensive deviation determination reference line information to the operator;
a step of the computer system acquiring the label data inputted into the screen by the operator; and
a step of, based on analysis of the reference data including the label data, the computer system updating the first comprehensive regression line information and the first comprehensive deviation determination reference line information to generate second comprehensive regression line information and second comprehensive deviation determination reference line information.

4. The data analysis method according to Claim 2, comprising:
a step of the computer system displaying, in the screen, a distribution diagram of evaluation parameters of the reaction process data and the comprehensive deviation determination reference line information; and
a step of, with respect to a first point in the reaction process data, the computer system estimating the possibility of an abnormality based on relation to the comprehensive deviation determination reference line information, displaying, in the screen, a first interface for inputting the label data with respect to the first point, and acquiring the label data inputted into the first interface by the operator.

5. The data analysis method according to Claim 4,
wherein the first interface embraces an abnormality present/absent field for inputting the presence/absence of the abnormality and an abnormality cause field for inputting the abnormality cause, and
in the first interface, the computer system displays, as an option, an initial value based on estimation of the presence/absence of an abnormality and an abnormality cause with respect to the first point in the abnormality present/absent field and the abnormality cause field.

6. The data analysis method according to Claim 4,
wherein the first interface embraces an abnormality present/absent field for inputting the presence/absence of the abnormality and an abnormality cause field for inputting the abnormality cause,
the data analysis method comprising:
a step of, in the abnormality cause field in the first interface, the computer system registering a new abnormality cause other than abnormality causes as a plurality of options based on an operation by the operator.

7. The data analysis method according to Claim 4, comprising:
a step of the computer system computing a nearby region with respect to the first point and, with respect to a second point which is located within the nearby region and for which the label data has not been inputted, displaying, in the screen, a second interface for inquiring about the presence/absence of an abnormality and an abnormality cause and prompting input of the label data.

8. The data analysis method according to Claim 7, comprising:
a step of, based on an operation by the operator with the second interface, the computer system displaying a third interface for inputting the label data with respect to the second point and acquiring the label data inputted into the third interface by the operator.

9. The data analysis method according to Claim 7,
wherein at the second interface, the computer system uses the same information as the contents of the label data with respect to the first point to display a message for inquiring about the presence/absence of the abnormality and the abnormality cause.

10. The data analysis method according to Claim 8,
wherein the third interface embraces an abnormality present/absent field for inputting the presence/absence of the abnormality and an abnormality cause field for inputting the abnormality cause, and
at the third interface, the computer system displays, as an option, an initial value identical with the contents of the label data with respect to the first point in the abnormality present/absent field and the abnormality cause field.

11. The data analysis method according to Claim 1,
wherein the computer system includes:
a first computer that is a computer provided in the automatic analysis system or a computer communicating with the automatic analysis system; and
a second computer that communicates with the first computer,
wherein the first step includes:
a step of the first computer acquiring, as the reference data, the apparatus information, the reagent information, and the reaction process data including the measurement data from each automatic analysis system of a plurality of automatic analysis systems; and
a step of the second computer acquiring the reference data from the first computer,
wherein the second step includes:
a step of the first computer acquiring the label data containing the presence/absence of an abnormality and an abnormality cause for each piece of the reaction process data inputted by the operator; and
a step of the second computer acquiring the label data from the first computer, and
the third step includes a step of, with respect to a distribution diagram of evaluation parameters of the reaction process data, based on analysis of the reference data including the label data, the second computer computing the comprehensive regression line information and the comprehensive deviation determination reference line information.

12. A data analysis system that performs analysis with respect to an automatic analysis system, comprising:
a computer system,
wherein the computer system
acquires, as reference data, apparatus information, reagent information, and reaction process data including measurement data from each automatic analysis system of a plurality of automatic analysis systems,
acquires label data containing the presence/absence of an abnormality and an abnormality cause for each piece of the reaction process data inputted by an operator, and
with respect a distribution diagram of evaluation parameters of the reaction process data, based on analysis of the reference data including the label data, computes comprehensive regression line information commonly applicable to a plurality of the automatic analysis systems and comprehensive deviation determination reference line information for determining deviation from the comprehensive regression line information.

13. A computer that performs analysis with respect to an automatic analysis system,
the computer acquiring, as reference data, apparatus information, reagent information, and reaction process data including measurement data from each automatic analysis system of a plurality of automatic analysis systems,
acquiring label data containing the presence/absence of an abnormality and an abnormality cause for each piece of the reaction process data inputted by an operator, and
with respect to a distribution diagram of evaluation parameters of the reaction process data, based on analysis of the reference data including the label data, computing comprehensive regression line information commonly applicable to a plurality of the automatic analysis systems and comprehensive deviation determination reference line information for determining deviation from the comprehensive regression line information.
